Europäisches Patentamt
European Patent Office
Office européen des brevets

(19)

(11) Veröffentlichungsnummer: **0 459 298 A2**

(12) **EUROPÄISCHE PATENTANMELDUNG**

(21) Anmeldenummer: **91108343.4**

(22) Anmeldetag: **23.05.91**

(51) Int. Cl.[5]: **A61K 31/13**, A61K 31/135, A61K 31/40, A61K 31/38

(30) Priorität: **26.05.90 DE 4017019**

(43) Veröffentlichungstag der Anmeldung: **04.12.91 Patentblatt 91/49**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kottmann, Hariolf, Dr.**
**Am Alten Birnbaum 6**
**W-6238 Hofheim am Taunus(DE)**
Erfinder: **Hänel, Heinz, Dr.**
**Taunusstrasse 148**
**W-6370 Oberursel(DE)**
Erfinder: **Kirsch, Reinhard, Dr.**
**Johannesallee 18**
**W-6230 Frankfurt am Main(DE)**

(54) **Verwendung von substituierten beta-Hydroxyethylaminen als potente Hemmstoffe der Exoenzyme von Pilzen.**

(57) Verbindungen der Formel III

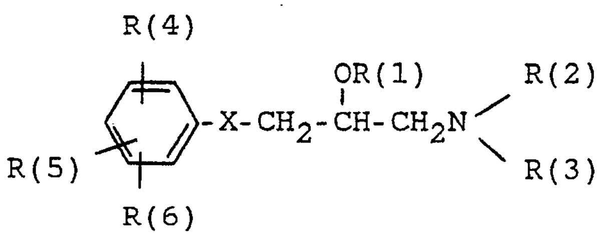

III,

die als $\beta$-Blocker bereits bekannt sind, dienen zur Vorbeugung gegen Pilzerkrankungen und zur Behandlung von Pilzerkrankungen.

A ist darin Alk(en)yl oder

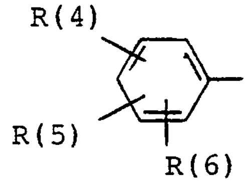

| | |
|---|---|
| X | ist Sauerstoff oderSchwefel |
| R(1) | ist darin Alk(en)yl oder Benzyl, |
| R(2) und R(3) | sind (Cyclo)Alk(en)yl, (Di)Phenyl(alk)(en)yl) oder gemeinsam eine $(CH_2)_m$-Kette, |
| R(4), R(5) und R(6) | können eine Vielzahl von bei derartigen $\beta$-Blockern üblichen Substituenten sein. |

EP 0 459 298 A2

Die Erfindung betrifft die Verwendung von substituierten β-Hydroxyethylaminen und von solchen Verbindungen enthaltende pharmazeutischen Zusammensetzungen als Pharmazeutika, insbesondere als Wachstumshemmer der pathogenen Phase dimorpher Hefezellen, als Antimykotika und Pflanzenschutzmittel, z.B. Fungizide und Wachstumsregulatoren, wobei die Verwendung der Verbindungen sowohl in der Prophylaxe als auch in der Therapie stattfinden kann.

Von einigen β-Hydroxyethylaminen, welche als Betablocker verwendet werden, ist bekannt, daß sie eine allerdings geringe Wirkung auf die Inhibierung der liposomalen Phospholipase A1 von Säugern besitzen. (vgl. K.Y. Hostetler et al., Biochemical Pharmacology 34, 521-524 (1985)). Wirkungsstärke und Verträglichkeit dieser Verbindungen sind jedoch nicht zufriedenstellend. (Beispiele sind Propranolol oder Metoprolol).

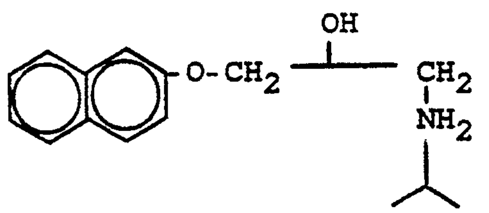

**Propranolol**

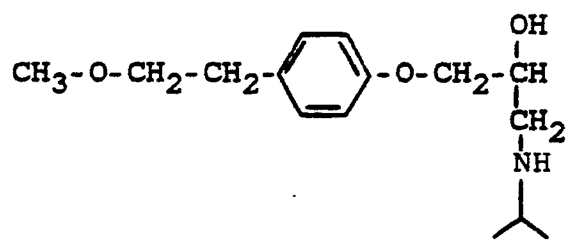

**Metoprolol**

Es wurde nun überraschenderweise gefunden, daß substituierte β-Hydroxyethylamine, in Abhängigkeit von der Struktur der jeweiligen Substituenten, potente Hemmstoffe der Exoenzyme (Pathogenitätsfaktoren) von Pilzen sind, fungizide sowie antimykotische Wirkung aufweisen und insbesondere hervorragende Wachstumshemmer der pathogenen Phase dimorpher Hefen sind.

Die Erfindung betrifft daher die Verwendung substituierter β-Hydroxyethylamine der Formel III

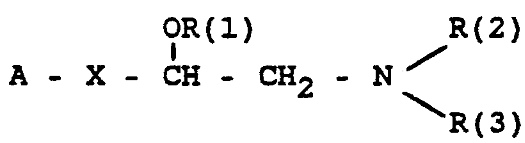

**(III)**

und von ihren Salzen mit pharmazeutisch akzeptablen Säuren sowie von diese Verbindungen III enthaltenden pharmazeutischen Zubereitungen zur Herstellung eines Medikaments zur Vorbeugung gegen und zur Behandlung von Pilzerkrankungen, wobei die Reste in Formel III folgende Bedeutung haben:

R(1) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), Benzyl, [unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl], C(O)-($C_1$-$C_8$)-Alkyl (geradkettig oder verzweigt), C(O)-phenyl

R(2) H, ($C_1$-$C_{18}$)-Alkyl (geradkettig oder verzweigt), ($C_1$-$C_6$)-Alkyl-oxy-($C_1$-$C_6$)-Alkyl (geradkettig oder verzweigt), ($C_1$-$C_6$)-Alkyl-oxy-phenyl (geradkettig oder verzweigt), ($C_2$-$C_{18}$)-Alkenyl (geradkettig oder verzweigt, ein- oder mehrfach ungesättigt), ($C_3$-$C_{12}$)-Cycloalkyl (mono-, bi- oder multicyclisch, wie Norbornyl, Adamantyl, Dekahydronaphthalinyl), Phenyl-($C_1$-$C_6$)-Alkyl (geradkettig oder verzweigt, wobei die Alkylkette unsubstituiert oder mit OH ein- oder zweifach substituiert ist), Phenyl-($C_2$-$C_6$)-Alkenyl (geradkettig oder verzweigt, ein- oder mehrfach ungesättigt), Diphenyl-($C_1$-$C_6$)-Alkyl (geradkettig oder verzweigt), Phenyl, wobei die Phenylsysteme unsubstituiert oder ein- oder mehrfach substituiert sind durch Substituenten aus der Gruppe F, Cl, Br, ($C_1$-$C_{10}$)-Alkyl (geradkettig oder verzweigt), ($C_3$-$C_{10}$)-Cycloalkyl, OH, SH, ($C_1$-$C_{10}$)-Alkoxy (geradkettig oder verzweigt), ($C_1$-$C_4$)-Alkylendioxy (geradkettig oder verzweigt), Dimethylaminoethoxy, ($C_1$-$C_4$)-Alkoxycarbonylmethoxy (geradkettig oder verzweigt), Phenoxy, Phenyl, Benzyl, Phenethyl, Thiophenyl, $C_nF_{2n+1}$ mit n gleich 1-6),

oder

R(2) ist ein Indol-3-yl-($C_1$-$C_4$)-Alkylrest (geradkettig oder verzweigt), Thienyl, Thienylmethyl, wobei der Thienylrest unsubstituiert oder mit F, Cl, ($C_1$-$C_4$)-Alkyl, O($C_1$-$C_4$)-Alkyl (geradkettig oder verzweigt) substituiert ist,

R(3) ist wie R(2) definiert, wobei R(2) und R(3) jeweils gleiche oder verschiedene Bedeutung aufweisen, oder

R(2) bildet mit R(3) eine Kette $-(CH_2)_m-$ mit m gleich 4-6, in welcher eine $CH_2$-Gruppe durch Sauerstoff, Schwefel oder Stickstoff ersetzt sein kann, wobei das zusätzliche N als weiteren Bindungspartner ein H-Atom, eine $CH_3$, Phenyl, Benzyl oder Phenethylgruppe trägt, und

A ($C_1$-$C_{18}$)-Alkyl (geradkettig oder verzweigt), ($C_2$-$C_{18}$)-Alkenyl (geradkettig oder verzweigt, ein- oder mehrfach ungesättigt) oder eine Gruppe der Formel II ist,

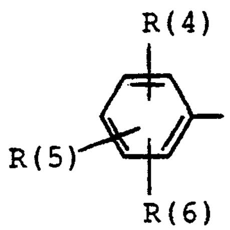

II

worin die Reste R(4), R(5) und R(6) die folgende Bedeutung haben:

R(4) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), ($C_3$-$C_{20}$)-Cycloalkyl,[mono-,bi- oder multicyclisch, unsubstituiert oder ein- oder zweifach mit ($C_1$-$C_4$)-Alkyl (geradkettig oder verzweigt), ($C_1$-$C_4$)-Alkoxy (geradkettig oder verzweigt), $C_nF_{2n+1}$ mit n gleich 1-4,F, Cl, Br, OH substituiert], Y-($C_1$-$C_{20}$)-Alkyl (geradkettig oder verzweigt), Y-($C_2$-$C_{18}$)-Alkenyl (geradkettig oder verzweigt ein- oder mehrfach ungesättigt), Y-($C_3$-$C_{20}$)-Cycloalkyl (mono-, bi- oder multicyclisch, unsubstituiert oder wie oben angegeben substituiert), Phenyl, Y-Phenyl, Phenyl-($C_1$-$C_4$)-Alkyl (geradkettig oder verzweigt), Phenyl-($C_1$-$C_4$)-Alkoxy (geradkettig oder verzweigt), Biphenylyl, F, Cl, Br, $C_nF_{2n+1}$ (mit n gleich 1-8), $CCl_3$, YH, Naphthyl, CN, $NO_2$, wobei die Phenylsysteme unsubstituiert sind oder ein- oder zweifach substituiert durch F, Cl, $CF_3$, ($C_1$-$C_4$)-Alkyl (geradkettig oder verzweigt), ($C_1$-$C_4$)-Alkoxy (geradkettig oder verzweigt),

mit Y gleich Sauerstoff oder Schwefel, SO, $SO_2$,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)$p-Kette mit p gleich 3 oder 4, und

R(6) H, ($C_1$-$C_{15}$)-Alkyl, (geradkettig oder verzweigt), ($C_2$-$C_{15}$)-Alkenyl (geradkettig oder verzweigt, ein- oder mehrfach ungesättigt), Y-($C_1$-$C_{15}$)-Alkyl (geradkettig oder verzweigt), Y-($C_2$-$C_{15}$)-Alkenyl (geradkettig oder verzweigt, ein- oder mehrfach ungesättigt), Phenyl, Y-Phenyl, Benzyl, Biphenylyl, F, Cl, Br, I, $C_nF_{2n+1}$ (mit n gleich 1-8), $CCl_3$, Naphthyl, YH,

X Sauerstoff, Schwefel.

Bevorzugt ist eine solche Verbindung von Verbindungen der Formel III in welcher die Reste folgende Bedeutung haben:

R(1) H, ($C_1$-$C_8$)-Alkyl (geradkettig oder verzweigt), ($C_3$-$C_8$)-Alkenyl (geradkettig oder versweigt, ein- oder zweifach ungesättigt), Benzyl [unsubstituiert oder ein- oder zweifach substituiert durch F, Cl, $CF_3$, ($C_1$-$C_4$)-Alkyl (geradkettig oder verzweigt), $OCH_3$], C(O)-($C_1$-$C_6$)-Alkyl (geradkettig oder verzweigt),

R(2) H, ($C_1$-$C_{16}$)-Alkyl (geradkettig oder versweigt), ($C_1$-$C_6$)-Alkyl-oxy-($C_1$-$C_6$)-Alkyl (geradkettig oder verzweigt), ($C_1$-$C_6$)-Alkyl-oxy-phenyl (geradkettig oder verzweigt), ($C_2$-$C_{16}$)-Alkenyl (geradkettig oder verzweigt, ein- oder mehrfach ungesättigt), ($C_3$-$C_{10}$)-Cycloalkyl, (mono-, bi- oder multicyclisch), Phenyl-($C_1$-$C_6$)-Alkyl (geradkettig oder verzweigt), Diphenyl-($C_1$-$C_6$)-Alkyl (geradkettig oder verzweigt), Phenyl, wobei die Phenylsysteme unsubstituiert oder ein- oder zweifach substituiert sind durch Substituenten aus der Gruppe F, Cl, ($C_1$-$C_4$)-Alkyl (geradkettig oder verzweigt), ($C_3$-$C_8$)-Cycloalkyl, ($C_1$-$C_8$)-Alkoxy (geradkettig oder verzweigt), ($C_1$-$C_2$)Alkylendioxy, ($C_1$-$C_4$)-Alkoxycarbonylmethoxy, Phenyl, Benzyl, Phenethyl, Thiophenyl,

oder

R(2) ist ein Indol-3-yl-($C_1$-$C_4$)-Alkylrest (geradkettig oder verzweigt), Thienylmethyl, wobei der Thienylrest unsubstituiert ist oder substituiert ist mit Cl, ($C_1$-$C_4$)-Alkyl (geradkettig oder verzweigt), O($C_1$-$C_4$)-Alkyl (geradkettig oder verzweigt),

R(3) ist wie R(2) definiert, wobei R(2) und R(3) jeweils gleiche oder verschiedene Bedeutung aufweisen, oder

R(2) bildet mit R(3) eine Kette $-(CH_2)_m-$ mit m gleich 4-6, in welcher eine $CH_2$-Gruppe durch Sauerstoff, Schwefel oder Stickstoff ersetzt sein kann, wobei das zusätzliche N als weiteren

Bindungspartner ein H-Atom, eine $CH_3$, Phenyl, Benzyl oder Phenethylgruppe trägt, und

A (C$_1$-C$_{16}$)-Alkyl (geradkettig oder verzweigt), (C$_3$-C$_{16}$)-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, ($C_1$-$C_{16}$)-Alkyl (geradkettig oder verzweigt), ($C_2$-$C_{16}$)-Alkenyl (geradkettig oder verzweigt, ein- oder mehrfach ungesättigt), ($C_3$-$C_{12}$)-Cycloalkyl [mono, bi- oder multicyclisch, unsubstituiert oder einfach mit ($C_1$-$C_4$)-Alkyl (geradkettig oder verzweigt) oder ($C_1$-$C_4$)-Alkoxy (geradkettig oder verzweigt) substituiert], Y-($C_1$-$C_{16}$)-Alkyl (geradkettig oder verzweigt), Y-($C_3$-$C_{16}$)-Alkenyl (geradkettig oder verzweigt, ein- oder mehrfach ungesättigt), Y-($C_3$-$C_{12}$)-Cycloalkyl (mono-, bi- oder multicyclisch, unsubstituiert oder wie oben angegeben subsituiert), Phenyl, Y-Phenyl, Phenyl-($C_1$-$C_2$)-Alkyl, Phenyl-($C_1$-$C_4$)-Alkoxy) (geradkettig oder verzweigt), Biphenylyl, F, Cl, $C_nF_{2n+1}$ (mit n gleich 1-4), $CCl_3$, Naphthyl, CN, wobei die Phenylsysteme unsubstituiert sind oder ein- oder zweifach durch F, Cl, $CF_3$, ($C_1$-$C_4$)-Alkyl, ($C_1$-$C_4$)-Alkoxy

mit Y gleich Sauerstoff oder Schwefel,

R(5) wie R(4) definierte wobei R(4) und R(5) gleich oder verschieden sind, oder

R(4) bildet mit R(5), für den Fall, daß die Substituenten an benachbarten Positionen am Phenylring gebunden sind, gemeinsam eine ($CH_2$)p-Kette mit p gleich 3 oder 4, und

R(6) H, ($C_1$-$C_{12}$)-Alkyl (geradkettig oder verzweigt), ($C_3$-$C_{12}$)-Alkenyl (geradkettig oder verzweigt, ein- oder mehrfach ungesättigt), Y-($C_1$-$C_{12}$)-Alkyl (geradkettig oder verzweigt), Y-($C_3$-$C_{12}$)-Alkenyl (geradkettig oder verzweigt, ein- oder mehrfach ungesättigt), Phenyl, Y-Phenyl, Benzyl, Biphenylyl, F, Cl, Br, $C_nF_{2n+1}$ (mit n gleich 1-4), $CCl_3$, Naphthyl,

X Sauerstoff, Schwefel.

Besonders bevorzugt ist eine Verwendung von Verbindungen der Formel III, wobei die Reste in Formel III folgende Bedeutung haben:

R(1) H, C(O)-($C_1$-$C_4$)-Alkyl (geradkettig oder verzweigt), Benzyl

R(2) H, ($C_1$-$C_{12}$)-Alkyl (geradkettig oder verzweigt), ($C_1$-$C_6$)-Alkyl-oxy-($C_1$-$C_6$)-Alkyl, ($C_3$-$C_{12}$)-Alkenyl (geradkettig oder verzweigt, ein- oder mehrfach ungesättigt), ($C_3$-$C_{10}$)-Cycloalkyl (mono-, bi- oder multicyclisch), Phenyl-($C_1$-$C_6$)-Alkyl (geradkettig oder verzweigt), Diphenyl-($C_1$-$C_6$)-Alkyl (geradkettig oder verzweigt), Phenyl, wobei die Phenylsysteme unsubstituiert oder ein- oder zweifach substituiert sind durch Substituenten aus der Gruppe $CF_3$, F, Cl, ($C_1$-$C_4$)-Alkyl (geradkettig oder verzweigt), ($C_1$-$C_8$)-Alkoxy (geradkettig oder verzweigt), ($C_1$-$C_2$)-Alkylendioxy, Phenyl, Benzyl, Phenethyl),

oder

R(2) ist ein Indol-3-yl($C_1$-$C_4$)-Alkylrest (geradkettig oder verzweigt), Thienylmethyl, wobei der Thienylrest unsubstituiert oder mit Cl, ($C_1$-$C_4$)-Alkyl, O($C_1$-$C_4$)-Alkyl substituiert ist

R(3) ist H,

R(2) bildet mit R(3) eine Kette -($CH_2$)$_m$- mit m gleich 4-6, in welcher eine $CH_2$-Gruppe durch Sauerstoff, Schwefel oder Stickstoff ersetzt sein kann, wobei N als weiteren Bindungspartner ein H-Atom, eine $CH_3$, Phenyl,Benzyl oder Phenethylgruppe trägt, und

A ($C_1$-$C_{12}$)-Alkyl (geradkettig oder verzweigt), ($C_3$-$C_{12}$)-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, ($C_1$-$C_{16}$)-Alkyl (geradkettig oder verzweigt), ($C_2$-$C_{12}$)-Alkenyl (geradkettig oder verzweigt, ein- oder mehrfach ungesättigt), ($C_3$-$C_{12}$)-Cycloakyl (mono-, bi- oder multicyclisch und unsubstituiert), Y-($C_1$-$C_{12}$)-Alkyl (geradkettig oder verzweigt), Y-($C_3$-$C_{12}$)-Alkenyl (geradkettig oder verzweigt, ein- oder mehrfach ungesättigt), Y-($C_3$-$C_{12}$)-Cycloalkyl (mono-, bi- oder multicyclisch und unsubstituiert), Phenyl, Y-Phenyl, Phenyl-($C_1$-$C_2$)-Alkyl, Phenyl-($C_1$-$C_4$)-Alkoxy (geradkettig oder verzweigt), Biphenyl, F, $Cl_4$ Naphthyl, wobei die Phenylsysteme unsubstituiert sind oder ein- oder zweifach substituiert durch F, Cl, $CF_3$, ($C_1$-$C_4$)-Alkyl (geradkettig oder verzweigt), ($C_1$-$C_4$)-Alkoxy (geradkettig oder verzweigt),

mit Y gleich Sauerstoff oder Schwefel und

R(5) wie R(4) definiert, wobei R(4) und R(5) gleich oder verschieden sind, und

R(6) H, ($C_1$-$C_{10}$)-Alkyl (geradkettig oder verzweigt), ($C_3$-$C_{10}$)-Alkenyl (geradkettig oder verzweigt, ein- oder mehrfach ungesättigt), Y-($C_1$-$C_{10}$)-Alkyl (geradkettig oder verzweigt, ein- oder mehrfach ungesättigt), Phenyl, Y-Phenyl, Benzyl, F, Cl, $CF_3$,

X Sauerstoff.

Ganz besonders bevorzugt ist die Verwendung von Verbindungen der Formel III, wobei die Reste in Formel III folgende Bedeutung haben:

R(1) H, C(O)-$CH_3$, Benzyl

R(2) H, ($C_1$-$C_{10}$)-Alkyl (geradkettig oder verzweigt), ($C_1$-$C_3$)-Alkyl-oxy-($C_1$-$C_3$)-Alkyl, ($C_3$-$C_{10}$)-Alkenyl (geradkettig oder verzweigt, ein- oder mehrfach ungesättigt), ($C_1$-$C_{10}$)-Cycloalkyl, (mono-, bi- oder multicyclisch), Phenyl-($C_1$-$C_4$)-Alkyl (geradkettig oder verzweigt), Diphenyl-($C_1$-$C_4$)-Alkyl (geradkettig oder verzweigt), Phenyl, wobei die Phenylsysteme unsubstutiert oder ein- oder zweifach substituiert sind durch Substituenten aus der Gruppe F, Cl, ($C_1$-$C_4$)-Alkyl (geradkettig oder verzweigt), ($C_1$-$C_6$)-Alkoxy (geradkettig oder verzweigt), ($C_1$-$C_2$)-Alkylendioxy, Phenyl, Benzyl, Phenethyl,

oder

R(2) ist ein Indol-3-yl-($C_1$-$C_4$)-Alkylrest (geradkettig oder verzweigt), Thienylmethyl, wobei der Thienylrest unsubstituiert oder mit Cl, ($C_1$-$C_4$)-Alkyl, O($C_1$-$C_4$)-Alkyl substituiert ist,

R(3) H

und

A ($C_1$-$C_{12}$)-Alkyl (geradkettig oder verzweigt), ($C_3$-$C_{10}$)-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, ($C_1$-$C_{12}$)-Alkyl (geradkettig oder verzweigt), ($C_2$-$C_{12}$)-Alkenyl (geradkettig oder verzweigt, ein- oder mehrfach ungesättigt), ($C_3$-$C_{10}$)-Cycloalkyl (mono-, bi- oder multicyclisch und unsubstituiert), Y-($C_1$-$C_{12}$)-Alkyl (geradkettig oder verzweigt), Y-($C_3$-$C_{12}$)-Alkenyl (geradkettig oder verzweigt, ein- oder mehrfach ungesättigt), Y-($C_3$-$C_{10}$)-Cycloalkyl (mono-, bi- oder multicyclisch und unsubstituiert), Phenyl, Y-Phenyl, Benyl, Benzyloxy, Biphenylyl, F, Cl, $CF_3$, wobei die Phenylsysteme unsubstituiert sind oder ein- oder zweifach substituiert durch F, Cl, $CF_3$, ($C_1$-$C_4$)-Alkyl (geradkettig oder verzweigt), ($C_1$-$C_4$)-Alkoxy (geradkettig oder verzweigt),

Y gleich Sauerstoff,

R(5) H, ($C_1$-$C_{12}$)-Alkyl (geradkettig oder verzweigt, ($C_2$-$C_{12}$)-Alkenyl (geradkettig oder verzweigt, ein- oder zweifach ungesättigt), ($C_3$-$C_6$)-Cycloalkyl, Y-($C_1$-$C_{12}$)-Alkyl (geradkettig oder verzweigt), Y-($C_3$-$C_6$)-Cycloalkyl,Phenyl, Y-Phenyl, Benzyl, F, Cl, $CF_3$,

R(6) gleich H,

X gleich Sauerstoff.

Es wurde nun überraschenderweise gefunden, daß substituierte $\beta$-Hydroxyethylamine, in Abhängigkeit von der Struktur der jeweiligen Substituenten, potente Hemmstoffe der Exoenzyme (Pathogenitätsfaktoren) von Pilzen sind, fungizide sowie antimykotische Wirkung aufweisen und insbesondere hervorragende Wachstumshemmer der pathogenen Phase dimorpher Hefen sind.

Die Erfindung betrifft daher die Verwendung von substituierten $\beta$-Hydroxyethylaminen sowie diese Verbindungen enthaltende pharmazeutische Zusammensetzungen der Formel I

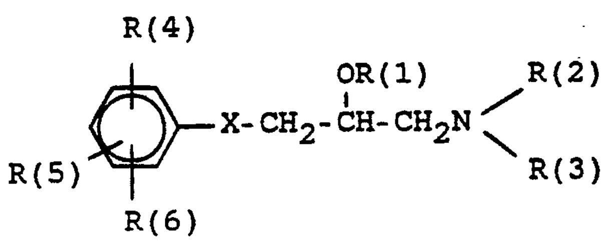

I,

zur Vorbeugung gegen und Behandlung von Pilzerkrankungen; in den Verbindungen bedeuten:

R(1) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), Benzyl (unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl)

R(2) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), ($C_3$-$C_{10}$)-Cycloalkyl, Phenyl-($C_1$-$C_6$) Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)Alkyl (geradkettig oder verzweigt), Phenyl, wobei das Phenylsystem unsubstituiert oder ein- oder mehrfach substituiert ist durch Substituenten aus der Gruppe F, Cl, Br, ($C_1$-$C_4$)-Alkyl (geradkettig oder verzweigt), ($C_3$-$C_8$)-Cycloalkyl, OH, SH, ($C_1$-$C_{10}$)-Alkoxy (geradkettig oder verzweigt), Phenyl, Benzyl, Phenethyl, Thiophenyl, $C_nF_{2n+1}$ mit n = 1-6),

R(3) wie R(2) definiert ist, wobei R(2) und R(3) jeweils gleiche oder verschiedene Bedeutung aufweisen,

oder

R(2) mit R(3) eine Kette $(CH_2)_m$- mit m gleich 4-6 bildet, in welcher eine $CH_2$-Gruppe durch O, S oder N ersetzt sein kann, wobei N als weiteren Bindungspartner ein H-Atom, eine $CH_3$, Phenyl-, Benzyl oder Phenethylgruppe trägt,

und

R(4) H, ($C_1$-$C_{15}$)-Alkyl (geradkettig oder verzweigt), ($C_2$-$C_{15}$)-Alkenyl (geradkettig oder verzweigt, ein- oder mehrfach ungesättigt), ($C_3$-$C_{20}$)-Cycloalkyl [mono-, bi- oder multicyclisch, unsubstituiert oder ein oder zweifach mit ($C_1$-$C_4$)-Alkyl (geradkettig oder verzweigt), ($C_1$-$C_4$)-Alkoxy (geradkettig oder verzweigt), $C_nF_{2n+1}$ (mit n gleich 1-4), F, Cl, Br, OH substituiert, wie z.B. Norbornyl, Adamantyl, Decahydronaphthalinyl]; Y-($C_1$-$C_{15}$)-Alkyl (geradkettig oder verzweigt), Y-($C_2$-$C_{15}$)-Alkenyl (geradkettig oder verzweigt, ein- oder mehrfach ungesättigt), Y-($C_3$-$C_{20}$)-Cycloalkyl (mono-, bi- oder multicyclisch, unsubstituiert oder wie oben angegeben substituiert), Phenyl, Y-Phenyl, Phenyl-($C_1$-$C_2$)-Alkyl, Biphenylyl, F, Cl, Br, I, $C_nF_{2n+1}$ (mit n gleich 1-8), $CCl_3$, YH, Naphthyl, CN, $NO_2$, mit Y gleich Sauerstoff oder Schwefel

und

R(5) wie R(4) definiert, wobei R(4) und R(5) gleich oder verschieden sind,

und

R(4) mit R(5), für den Fall, daß die Substituenten an benachbarten Positionen am Phenylring gebunden sind, gemeinsam eine $(CH_2)_p$-Kette mit p gleich 3 oder 4 bilden können,

und

R(6) H, ($C_1$-$C_{15}$)-Alkyl (geradkettig oder verzweigt), ($C_2$-$C_{10}$)-Alkenyl (geradkettig oder verzweigt, ein- oder mehrfach ungesättigt), Y-($C_1$-$C_{15}$)-Alkyl (geradkettig oder verzweigt), Y-($C_2$-$C_{15}$)-Alkenyl (geradkettig oder verzweigt, ein oder mehrfach ungesättigt), Phenyl, Y-Phenyl, Benzyl, Biphenylyl, F, Cl, Br, I, $C_nF_{2n+1}$ (mit n gleich 1-8), $CCl_3$, Naphthyl, YH

Y = Sauerstoff, Schwefel

X = Sauerstoff, Schwefel, SO, $SO_2$,

und ihre Salze mit pharmazeutisch akzeptablen ,Säuren.

Bevorzugt sind Verbindungen des Typs I, in denen die Substituenten folgende Bedeutung besitzen:

R(1) H, ($C_1$-$C_8$)-Alkyl (geradkettig oder verzweigt),($C_3$-$C_{10}$)-Alkenyl (geradkettig oder verzweigt, ein- oder mehr ungesättigt), Benzyl (unsubstituiert oder einfach oder zweifach substituiert durch F, Cl, $CF_3$, ($C_1$-$C_4$)-Alkyl (geradkettig oder verzweigt), $OCH_3$,

R(2) H, ($C_1$-$C_8$)-Alkyl (geradkettig oder verzweigt), ($C_3$-$C_{10}$)-Alkenyl (geradkettig oder verzweigt, ein- oder mehrfach ungesättigt), Phenyl-($C_1$-$C_6$)-Alkyl (geradkettig oder verzweigt), Diphenyl-($C_1$-$C_6$)-Alkyl (geradkettig oder verzweigt), Phenyl, wobei das Phenylsystem unsubstituiert oder ein- oder zweifach substituiert ist durch Substituenten aus der Gruppe OH, F, Cl, Br, ($C_1$-$C_4$)-Alkyl (geradkettig oder verzweigt), ($C_1$-$C_4$)-Alkoxy (geradkettig oder verzweigt), Phenyl, Benzyl, Phenethyl,

R(3) wie R(2) definiert ist, wobei R(2) und R(3) jeweils gleiche oder verschiedene Bedeutung aufweisen,

oder

R(2) mit R(3) eine Kette -$(CH_2)_m$- mit m gleich 4-6 bildet, in welcher eine $CH_2$-Gruppe durch ein Sauerstoff oder Stickstoff-Atom ersetzt sein kann, wobei N als weiteren Bindungspartner ein H-Atom, eine $CH_3$, Phenyl-, Benzyl oder Phenethylgruppe trägt,

und

R(4) H, ($C_1$-$C_{15}$)-Alkyl (geradkettig oder verzweigt), ($C_2$-$C_{15}$)-Alkenyl (geradkettig oder verzweigt, ein- oder mehrfach ungesättigt), ($C_3$-$C_{15}$)-Cycloalkyl [mono-, bi- oder multicyclisch, unsubstituiert oder einfach mit ($C_1$-$C_4$)-Alkyl (geradkettig oder verzweigt), ($C_1$-$C_4$)-Alkoxy (geradkettig oder verzweigt), substituiert, wie z.B. Norbornyl, Adamantyl, Decahydronaphthalinyl], Y-($C_1$-$C_{10}$)-Alkyl (geradkettig oder verzweigt), Y-($C_2$-$C_{10}$)-Alkenyl (geradkettig oder verzweigt, ein- oder mehrfach ungesättigt), Y-($C_3$-$C_{15}$)-Cycloalkyl (mono-, bi- oder multicyclisch, unsubstituiert oder wie oben angegeben substituiert), Phenyl, Y-Phenyl, Phenyl-($C_1$-$C_2$)-Alkyl, Biphenylyl, F, Cl, $C_nF_{2n+1}$ (mit n gleich 1-4), $CCl_3$, Naphthyl, CN, mit Y gleich Sauerstoff oder Schwefel,

und

R(5) wie R(4) definiert ist, wobei R(4) und R(5) gleich oder verschieden sind,

und

R(4) mit R(5) für den Fall, daß die Substituenten an benachbarten Positionen am Phenylring gebunden sind, gemeinsam eine $(CH_2)_p$-Kette mit p gleich 3 oder 4 bilden können,

und

R(6) H, $(C_1-C_{10})$-Alkyl (geradkettig oder verzweigt), Y-$(C_1-C_{10})$-Alkyl (geradkettig oder verzweigt), Y-$(C_2-C_{10})$-Alkenyl (geradkettig oder verzweigt, ein- oder zweifach ungesättigt), Phenyl, Benzyl, Biphenylyl, E, Cl, Br, $C_nF_{2n+1}$ (mit n gleich 1-4), $CCl_3$, Naphthyl,

Y = Sauerstoff, Schwefel

X = Sauerstoff, Schwefel.

Besonders bevorzugt sind Verbindungen I, bei denen die Substituenten folgende Bedeutung haben:

R(1) H, $(C_1-C_8)$-Alkyl (geradkettig oder verzweigt), Benzyl (unsubstituiert oder ein- oder zweifach substituiert durch F, Cl, $CF_3$, $(C_1-C_4)$-Alkyl (geradkettig oder verzweigt), $OCH_3$,

R(2) H, $(C_1-C_8)$-Alkyl (geradkettig oder verzweigt), $(C_3-C_{10})$-Alkenyl (geradkettig oder verzweigt, ein- oder zweifach ungesättigt), Phenyl-$(C_1-C_6)$-Alkyl (geradkettig oder verzweigt), Diphenyl-$(C_1-C_6)$-Alkyl (geradkettig oder verzweigt), Phenyl, wobei das Phenylsystem unsubstituiert oder ein- oder zweifach substituiert ist durch Substituenten aus der Gruppe OH, F, Cl, Br, $(C_1-C_4)$-Alkyl (geradkettig oder verzweigt), $(C_1-C_4)$-Alkoxy (geradkettig oder verzweigt), Phenyl, Benzyl,

R(3) H

oder

R(2) gemeinsam mit R(3) eine Kette $-(CH_2)_m-$ mit m gleich 4-5 bildet, in welcher eine $CH_2$-Gruppe durch ein Sauerstoff- oder Stickstoff-Atom ersetzt sein kann, wobei der Stickstoff als weiteren Bindungspartner ein H-Atom, eine $CH_3$, Phenyl, Benzyl oder Phenethylgruppe trägt,

und

R(4) H, $(C_1-C_{10})$-Alkyl (geradkettig oder verzweigt), $(C_2-C_{10})$-Alkenyl (geradkettig oder verzweigt, ein- oder mehrfach ungesättigt), $(C_3-C_{15})$-Cycloalkyl [mono-, bi- oder multicyclisch, wie z.B. Norbornyl, Adamantyl, Decahydronaphthalinyl)], Y-$(C_1-C_{10})$-Alkyl (geradkettig oder verzweigt), Y-$(C_2-C_{10})$-Alkenyl (geradkettig oder verzweigt, ein- oder mehrfach ungesättigt), Y-$(C_3-C_{15})$-Cycloalkyl (mono-, bi- oder multicyclisch), Phenyl, Y-Phenyl, Benzyl, Biphenylyl, F, Cl, $C_nF_{2n+1}$ (mit n gleich 1-4), Naphthyl, CN, mit Y gleich Stickstoff oder Schwefel,

und

R(5) wie R(4) definiert ist, wobei R(4) und R(5) gleich oder verschieden sind,

und

R(6) H, $(C_1-C_4)$-Alkyl (geradkettig oder verzweigt), Y-$(C_1-C_4)$-Alkyl (geradkettig oder verzweigt), F, Cl, $CF_3$

Y = Sauerstoff, Schwefel,

X = Sauerstoff.

Ganz besonders bevorzugt sind Verbindungen I, bei denen die Substituenten folgende Bedeutung haben:

R(1) H, Benzyl,

R(2) H, $(C_1-C_8)$-Alkyl (geradkettig oder verzweigt), Phenyl-$(C_1-C_4)$-Alkyl (geradkettig oder verzweigt), wobei das Phenylsystem unsubstituiert oder einfach substituiert ist durch F, Cl, $(C_1-C_4)$-Alkyl (geradkettig oder verzweigt), $(C_1-C_4)$-Alkoxy (geradkettig oder verzweigt),

R(3) H,

R(4) H, $(C_1-C_{10})$-Alkyl (geradkettig oder verzweigt), $(C_2-C_{10})$-Alkenyl (geradkettig oder verzweigt, ein- oder mehrfach ungesättigt), $(C_3-C_{15})$-Cycloalkyl [mono-, bi- oder multicyclisch (wie z.B. Norbornyl, Adamantyl, Decahydronaphthalinyl)], Y-$(C_1-C_{10})$-Alkyl (geradkettig oder verzweigt), Y-$(C_3-C_{15})$-Cycloalkyl (mono-, bi- oder multicyclisch), Phenyl, Y-Phenyl, Benzyl, Biphenylyl, F, Cl, $C_nF_{2n+1}$ (mit n gleich 1-4),

mit Y gleich Sauerstoff

und

R(5) H, $(C_1-C_6)$-Alkyl (geradkettig oder verzweigt), $(C_2-C_6)$-Alkenyl (geradkettig oder verzweigt, ein- oder mehrfach ungesättigt), $(C_3-C_6)$-Cycloalkyl Y-$(C_1-C_6)$-Alkyl (geradkettig oder verzweigt), Y-$(C_3-C_6)$-Cycloalkyl, Phenyl, Y-Phenyl, Benzyl, F, Cl, $CF_3$ mit Y gleich Sauerstoff,

R(6) H,

X gleich Sauerstoff.

Alkyl oder Alkenyl bedeuten in allen Fällen sowohl die geradkettigen als auch die verzweigten Gruppen.

Die Verbindungen I werden zum Beispiel nach US 4 191 765 (HOE 76/F 115) hergestellt. Die als Ausgangsmaterialien verwendeten Verbindungen sind entweder käuflich oder werden nach bekannten Verfahren erhalten.

Die Erfindung betrifft die Verwendung der Verbindungen I in Form der freien Base oder eines Säureadditionssalzes als potente Hemmstoffe der Exoenzyme von Pilzen.

Beispiele pharmazeutisch akzeptabler salzbildender Säuren sind anorganische Säuren wie Salzsäure, Bromwasserstoffsäure, Jodwasserstoffsäure, Schwefelsäure, Phosphorsäure oder Salpetersäure oder organische Säuren wie Malonsäure-, Oxalsäure, Gluconsäure, Camphersulfonsäure, Benzolsulfonsäure, Essigsäure, Propionsäure, p-Toluolsulfonsäure oder Salicylsäure.

Die Verbindungen I weisen mindestens ein asymmetrisches C-Atom auf und können daher als Enantiomere und Diasteromere auftreten. Die Erfindung umfaßt sowohl die Verwendung der reinen Isomeren als auch von deren Gemischen. Gemische von Diastereomeren können nach gebräuchlichen Methoden, z.B. durch selektive Kristallisation aus geeigneten Lösungsmitteln oder durch Chromatographie an Kieselgel oder Aluminiumoxid in die Komponenten aufgetrennt werden.

Racemate können nach üblichen Methoden in die Enantiomeren aufgetrennt werden, z.B. durch Salzbildung mit einer optisch aktiven Säure, Trennung der diastereomeren Salze und Freisetzung der reinen Enantiomeren mittels einer Base. Darüber hinaus lassen sich die Enantiomeren auch mittels Chromatographie an chiralen Phasen oder auf enzymatischem Wege trennen.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung gegen und Behandlung von Pilzinfektionen beim Menschen und bei verschiedenen Säugetierarten.

Die Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Hierbei wird von der Hefe Candida albicans insbesondere das Exoenzymsystem dergestalt beeinflußt, daß die Pathogenität der Erreger deutlich absinkt. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:
Dermatomykosen und Systemmykosen durch Trichophyton mentagrophytes und andere Trichophytonarten, Mikrosporenarten, Epidermophyton floccosum, und biphasische Pilze sowie Schimmelpilze hervorgerufen. Insbesondere werden tiefe Mykosen, die durch Candida albicans hervorgerufen werden, günstig beeinflußt, da hierbei ein Eindringen der Pilze in die Wirtszelle verhindert bzw. erschwert wird.

Als Indikationsgebiete in der Tiermedizin können beispielsweise aufgeführt werden:
Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere Wirkstoffe enthalten oder die aus einem oder mehreren erfindungsgemäß verwendeten Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Ein Hemmstoff für die unterschiedlichen Phospholipasen von Candida albicans muß im Patienten überall dort in hinreichenden Konzentrationen vorliegen, wo der Pilz die Parenchyme besiedeln kann. Dieser Umstand setzt voraus, daß die entsprechenden Substanzen in einer Konzentration verabreicht werden müssen, die sich zuvor in Tierexperimenten als wirksam erwiesen hat.

Bei den schweren Krankheitsbildern der tiefen Candidose befinden sich die Patienten meist in einem sehr schlechten Allgemeinzustand. Hohes Fieber und weitere Erkrankungen sind häufig anzutreffen. Bei den Dosierungsvorgaben muß zwischen der prophylaktischen Gabe und der Therapie im nachgewiesenen Infektionsfall unterschieden werden. Bei der Prophylaxe kann von einem besseren Allgemeinzustand der Patienten ausgegangen werden, der eine orale Verabreichung ermöglicht. Hierbei können Tabletten, Lösungen, Gele oder Trockensaft zum Einsatz kommen. Bei den Formen mit nachgewiesenen tiefen Candidosen muß oft davon ausgegangen werden, daß eine geregelte orale Aufnahme der Wirkstoffe nicht immer gewährleistet ist. Hierfür kommen dann parenterale Anwendungsformen in Frage. Im Ausnahmefall kann auch an eine subcutane Verabreichung gedacht werden.

Als Kandidaten für eine Prophylaxe kommen in erster Linie immunkomprimierte Patienten in Frage, die durch entsprechende medikamentöse Belastung oder durch körpereigene Immunprobleme in dieser Situation sind. Dies sind insbesondere Transplantationspatienten, Zuckerkranke und/oder adipöse Patienten, AIDS-Patienten, unter Chemotherapie stehende Patienten, Langzeitbeatmete usw..

Die Verbindungen zeigen Hemmungen der Phospholipase von Candida albicans, die weit unter der in vitro festgestellten minimalen inhibitorischen Konzentrationen der Wirkstoffe gegenüber Candida albicans liegen. Daher kann die Dosierung im Regelfall unter derjenigen liegen, die für eine reine antimykotische Therapie nötig wäre.

Die Wirkung im Patienten beruht darauf, daß die Wirkstoffe bei den Candida Zellen, die sich in der Nähe der Parenchyme befinden, zwei unterschiedliche Effekte auslöst. Zum einen wird die Adhäsion der Hefezellen an die Körperzellen verhindert und zum anderen wird Candida albicans daran gehindert, die Körperzellen mit Keimschläuchen zu penetrieren.

Durch dieses duale Wirkungskonzept kann die Hefe ihre Pathogenität nicht zur vollen Ausprägung bringen. Allerdings muß erwähnt werden, das Candida albicans außer den Phospholipasen noch weitere Pathomechanismen wie z.B. die Protease besitzt. Die Anheftung an körpereigene Zellen ist jedoch der primäre Schritt zur Penetration. Da diese Anheftung durch Phospholipasehemmer verhindert wird, können die anderen Pathomechanismen nicht voll zur Geltung kommen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die prophylaktisch und therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäß verwendeten Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffs oder der Wirkstoffe mit dem Trägerstoff oder den Trägerstoffen.

Zur vorliegenden Erfindung gehört die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäß verwendeten Wirkstoffe in Gesamtmengen von mindestens etwa 0,05, vorzugsweise 0,1 , insbesondere 0,5 mg/kg Körpergewicht bis höchstens etwa 200, vorzugsweise bis 100, insbesondere bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen, bei tiefen Mykosen jedoch über wesentlich längere Zeiträume (bis zu 6 Wochen) verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Verbindungen der Formel I sind auch als Biozide wirksam. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen

Fungiziden nicht mehr wirksam bekämpft werden können. Das Wirkungsspektrum der Verbindungen I erfaßt eine Vielzahl verschiedener phytopathogener Pilze, wie z.B. Botrytis cinerea, Echte Mehltau-Arten, Drechslera teres, Venturia inaequalis, Cerospora beticola, Phytophthora infestans, Plasmopara viticola, verschiedene Rostarten, vor allem aber Plasmopara viticola, im Obst-, Gemüse-, Getreide- und Zierpflanzenbau.

Die Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem

Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinapthylmethan-6,6'-di-sulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
alkylarylsulfonsaure Calciumsalze, wie Ca-dodecylbenzolsulfonat, oder nicht ionische Emulgatoren wie Fettsäurepolyglykolester, Alkylarylpolyglykolether, Fettalkoholpolyglykolether, Propylenoxid- Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-% besteht aus üblichen Formulierungsbestandteilen. Bei emulgierbaren Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:
Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

Als Beispiel für die Hemmung der pathogenen Phase dimorpher Hefezellen werden Ergebnisse eines in-vitro-Enzymtests angeführt, in welchen die prozentuale Hemmung freigesetzter Exoenzyme, insbesondere freigesetzter Lysophospholipase (Phospholipase B), als Maß für die Wirksamkeit bestimmt wird.

Zur Feststellung der Enzymhemmung wurde eine Suspension von Candida albicans Blastokonidien (Stamm 200/175), wobei die Keimdichte photometrisch auf eine Extinktion von 0,5 (500 nm) eingestellt war, mit Präparatelösung bzw. zur Kontrolle mit Lösungsmittellösung vermischt und zwar

a) 100 µl Präparatelösung (Suspension)
+ 900 µl Keimsuspension
b) 100 µl Lösungsmittel

+ 900 µl Keimsuspension

Jeweils 5 µl der 30 min bei 21 °C inkubierten Blastokonidiensuspension wurden auf eine Agarplatte (Sabourand Agar mit Zusatz von 8 % Eigelb, 1 M NaCl, 5 mM $CaCl_2$) aufgetropft.

Die so beimpfte Platte wurde 3 Tage bei 37 °C bebrütet.

Die Auswertung erfolgte derart, daß

1. der Durchmesser (mm) der Candida albicans-Kolonie (behandelt und unbehandelt) sowie
2. der Gesamtdurchmesser von Kolonie und Trübungshof, welcher durch Exoenzyme verursacht wurde (behandelt und unbehandelt) bestimmt wurde.

Aus der Bestimmung des Quotienten von Präparate- und Kontrollgruppe ergab sich ein Wert, der als Maß für die Enzymaktivität anzusehen war.

Wie aus Tabelle 1 ersichtlich, hemmen die erfindungsgemäß verwendeten Verbindungen die freigesetzten Exoenzyme wesentlich stärker als Propranolol

Propanol wird in der Literatur (Pappu A.S. et al. in Biochem. Pharmacol. 34, 521-24, 1985) als wirksamste Substanz beschrieben, die gegenüber Phospholipase aus Leberzellen in einem entsprechenden in-vitro-Test Hemmwirkung zeigte.

Der Stand der Technik wird durch die erfindungsgemäß verwendeten Verbindungen I überraschenderweise deutlich übertroffen.

**Tabelle 1**

Prozentuale Hemmung der freigesetzten Exoenzyme von Candida albicans in vitro

| Präparat | Konzentration (µm/ml) | % Hemmung der Phospholipase |
|---|---|---|
| 1.1 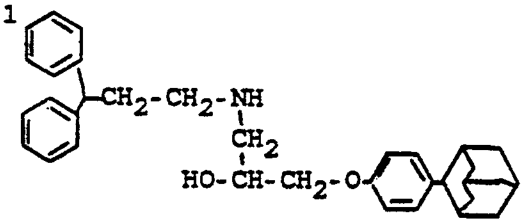 | 5 | 62,5 |
| 1.2 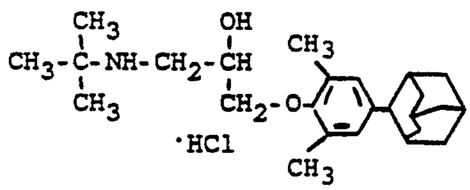 | 5 | 50 |
| 1.3 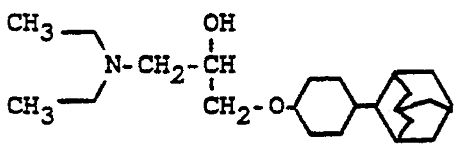 | 10 | 42,8 |
| 1.4 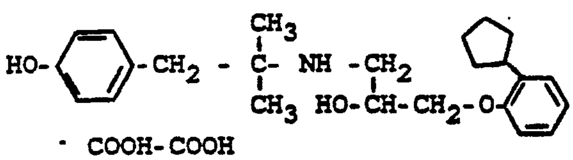 | 50 | 100 |

**Fortsetzung Tabelle 1**

| Präparat | | Konzentration (µm/ml) | % Hemmung der Phospholipase |
|---|---|---|---|
| 1.5 | 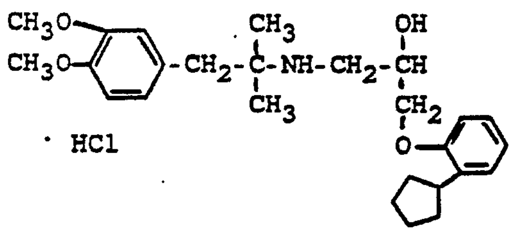 · HCl | 50 | 50 |
| 1.6 | 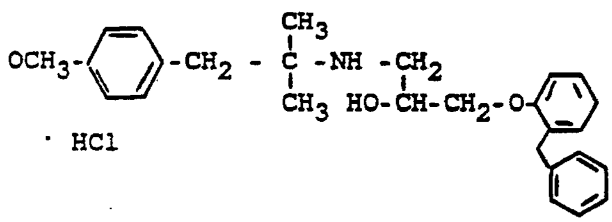 · HCl | 10 | 87.5 |
| 1.7 | 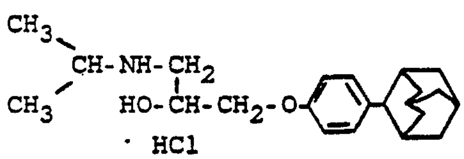 · HCl | 10 | 50 |
| 1.8 | 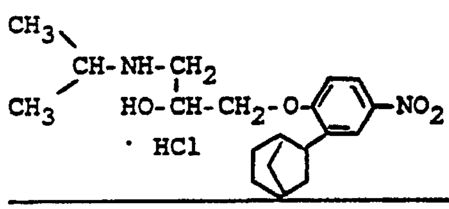 · HCl | 50 | 75 |
| | | 10 | 25 |
| 1.9 | 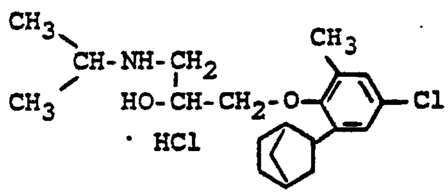 · HCl | 10 | 75 |

**Fortsetzung Tabelle 1**

| Präparat | Konzentration (µm/ml) | % Hemmung der Phospholipase |
|---|---|---|
| 1.10<br>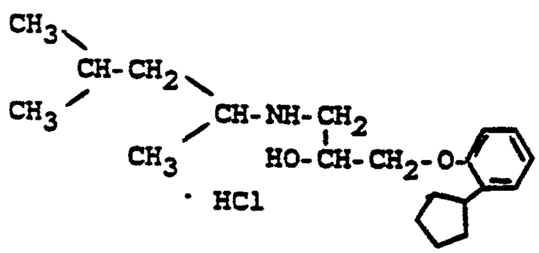 | 10 | 75 |
| 1.11<br>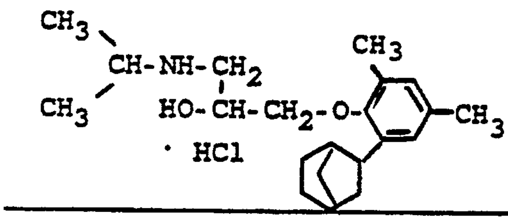 | 50 | 40 |
| 1.12<br>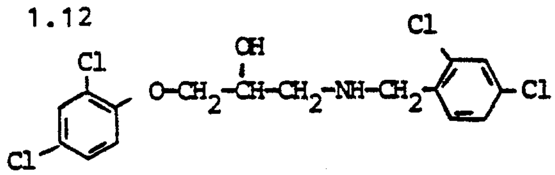 | 100<br>50 | 100<br>100 |
| 1.13<br>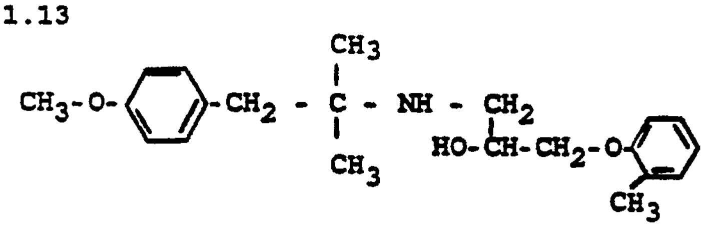 | 50<br>10 | 100<br>30 |
| 1.14<br>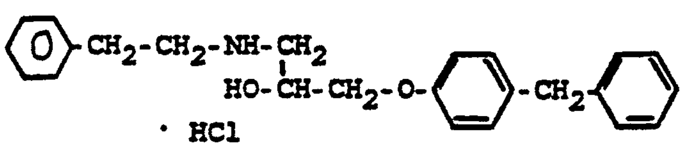 | 50<br>10 | 100<br>33 |

**Fortsetzung Tabelle 1**

| Präparat | Konzentration (µg/ml) | % Hemmung der Phospholipase |
|---|---|---|
| 1.15 | 50 | 86 |
| | 10 | 29 |
| 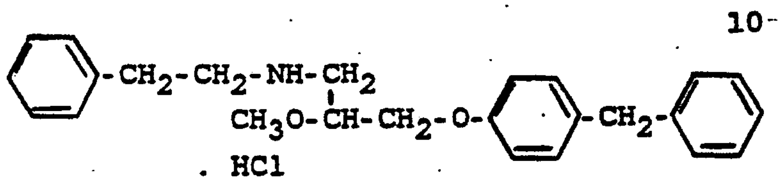 | | |
| 1.16 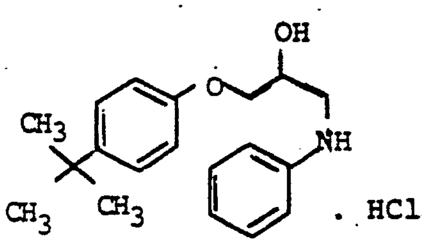 | 100 | 100 |
| | 50 | 100 |
| | 10 | 50 |
| 1.17 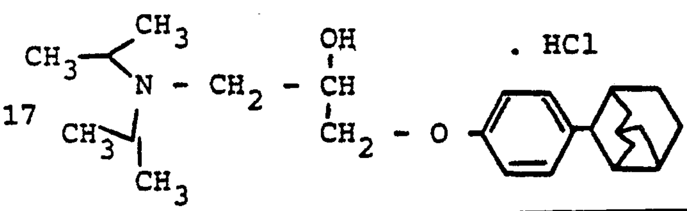 | 100 | 100 |
| | 50 | 100 |
| | 10 | 92,9 |
| 1.18 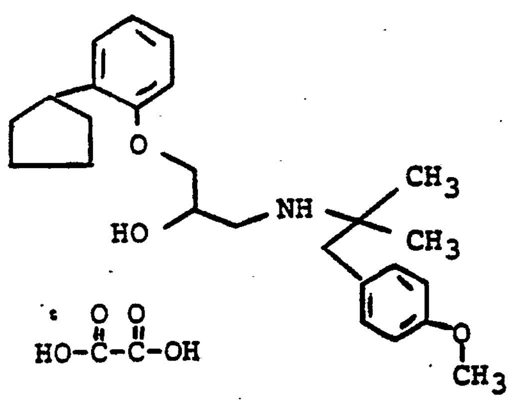 | 50 | 91 |
| | 10 | 14,3 |

Fortsetzung Tabelle 1

| Präparat | Konzentration (µm/ml) | % Hemmung der Phospholipase |
|---|---|---|
| 1.19 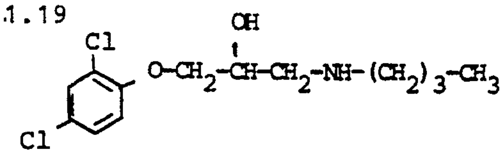 | 100<br>50 | 100<br>87,5 |
| 1.20 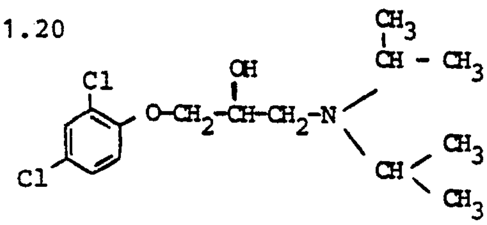 | 100<br>50 | 100<br>30 |
| 1.21 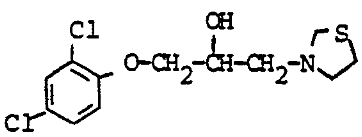 | 100<br>50<br>10 | 100<br>44,4<br>25 |
| 1.22 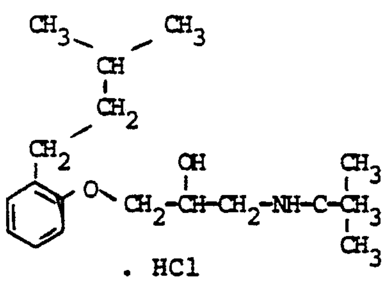 . HCl | 100<br>50 | 100<br>100 |
| 1.23 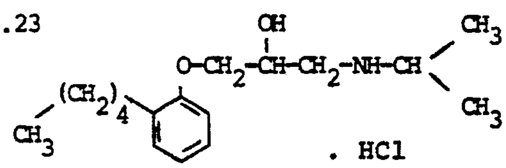 . HCl | 100<br>50 | 100<br>100 |
| 1.24 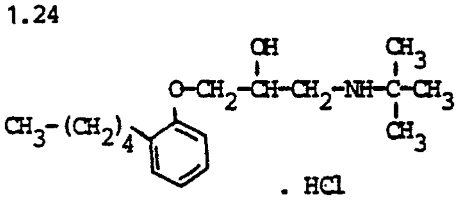 . HCl | 100<br>50 | 100<br>100 |
| Propranolol | 100 | 30 |

Fortsetzung Tabelle 1

| Präparat | Konzentration (µm/ml) | % Hemmung der Phospholipase |
|---|---|---|
| 1.25 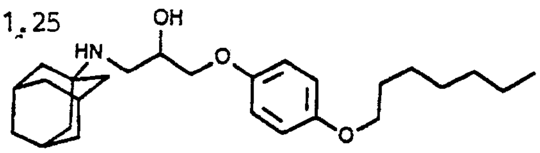 | 50 | 100 |
| | 10 | 100 |
| | 5 | 20 |
| 1.26 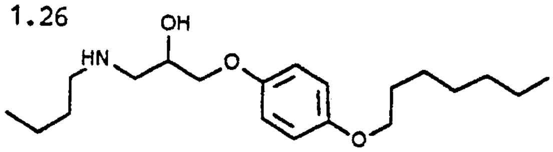 | 50 | 100 |
| | 10 | 100 |
| | 5 | 22 |
| 1.27 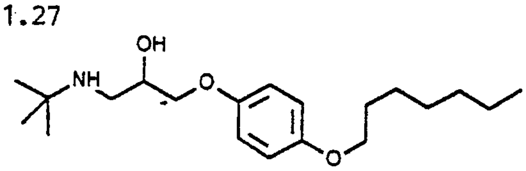 | 50 | 100 |
| | 10 | 100 |
| | 5 | 30 |
| 1.28 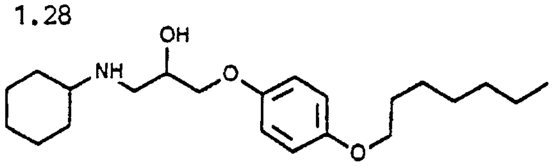 | 50 | 100 |
| | 10 | 100 |
| | 5 | 45 |
| 1.29 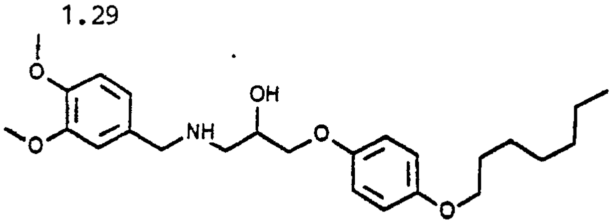 | 10 | 100 |
| | 5 | 100 |
| | 1 | 10 |
| 1.30 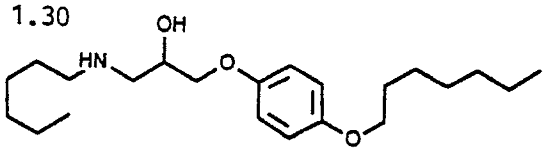 | 10 | 100 |
| | 5 | 100 |
| | 1 | 0 |

Fortsetzung Tabelle 1

| Präparat | Konzentration (µm/ml) | % Hemmung der Phospholipase |
|---|---|---|
| 1.31 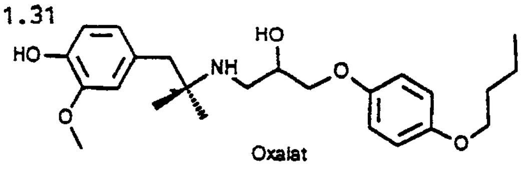 Oxalat | 100 | 100 |
| | 50 | 67 |
| | 10 | 20 |
| 1.32 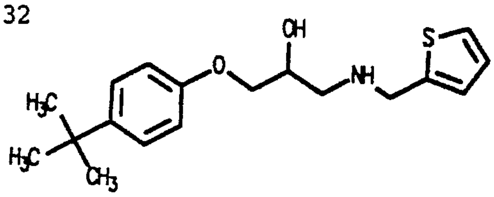 | 100 | 100 |
| | 50 | 100 |
| | 10 | 100 |
| 1.33 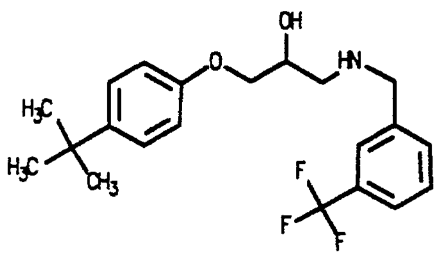 | 100 | 100 |
| | 50 | 100 |
| | 10 | 10 |
| 1.34 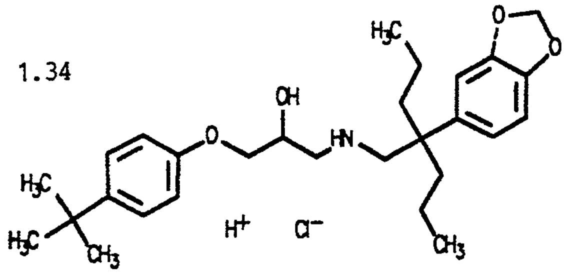 | 100 | 100 |
| | 50 | 100 |
| | 10 | 100 |
| | 5 | 100 |
| 1.35 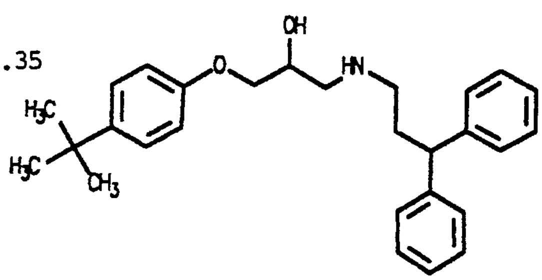 | 100 | 100 |
| | 50 | 100 |
| | 10 | 33 |

Fortsetzung Tabelle 1

| Präparat | Konzentration (µm/ml) | % Hemmung der Phospholipase |
|---|---|---|
| 1.36 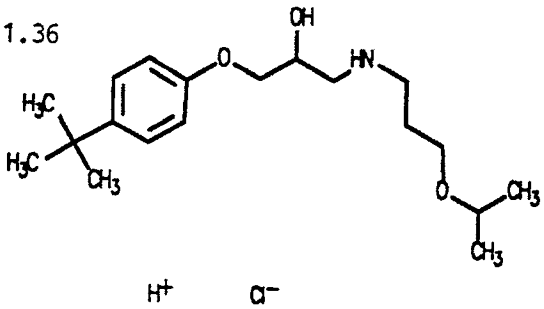 | 100 | 100 |
| | 50 | 100 |
| | 10 | 100 |
| | 5 | 8 |
| 1.37 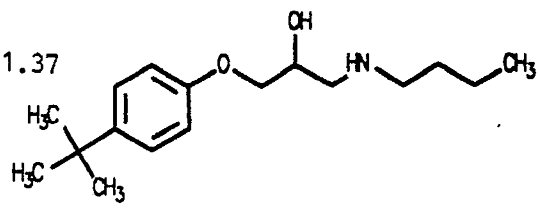 | 100 | 100 |
| | 50 | 100 |
| 1.38 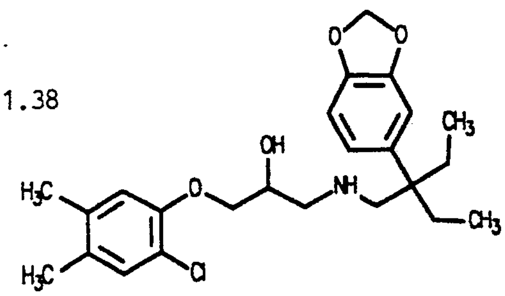 | 100 | 100 |
| | 50 | 100 |
| 1.39 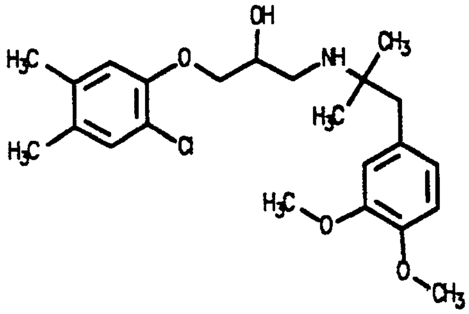 | 100 | 100 |
| | 50 | 100 |

Fortsetzung Tabelle 1

| Präparat | Konzentration (µm/ml) | % Hemmung der Phospholipase |
|---|---|---|
| 1.40 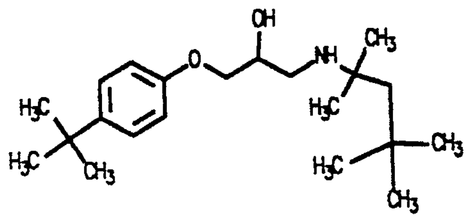 | 100 | 100 |
| | 50 | 100 |
| | 10 | 100 |
| | 5 | 17 |
| 1.41 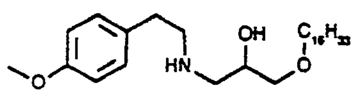 | 100 | 100 |
| | 50 | 100 |
| | 10 | 50 |
| 1.42 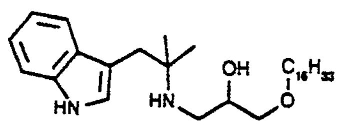 | 100 | 100 |
| | 50 | 100 |
| 1.43 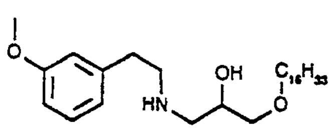 | 10 | 100 |
| | 5 | 100 |
| 1.44 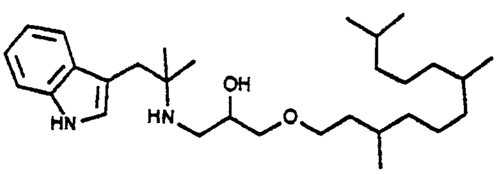 | 100 | 100 |
| | 50 | 100 |
| | 10 | 45 |
| 1.45 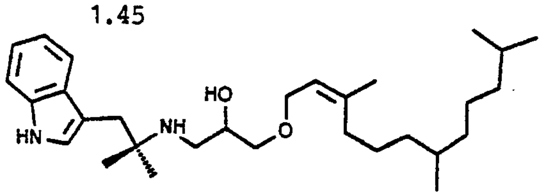 | 100 | 100 |
| | 50 | 100 |
| | 10 | 10 |

Fortsetzung Tabelle 1

| Präparat | Konzentration (µm/ml) | % Hemmung der Phospholipase |
|---|---|---|
| 1.46 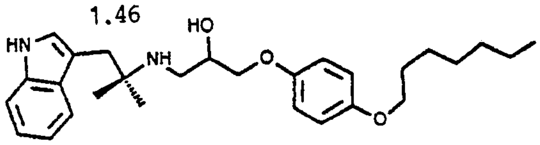 | 10 | 100 |
| | 5 | 100 |
| 1.47 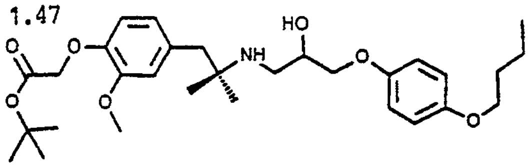 | 100 | 100 |
| | 50 | 50 |
| 1.48 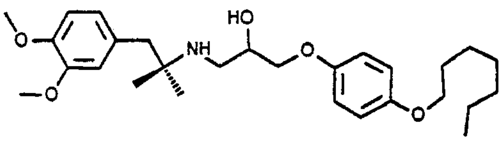 | 10 | 100 |
| | 5 | 100 |
| | 1 | 10 |
| 1.49 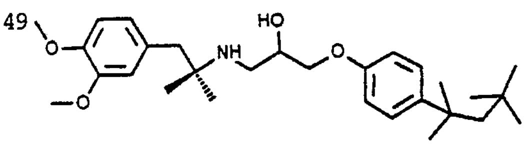 | 100 | 100 |
| | 50 | 100 |
| | 10 | 11 |
| 1.50 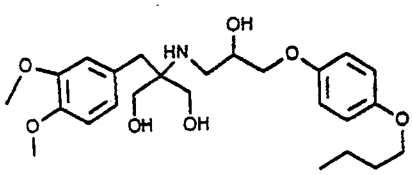 | 100 | 56 |
| | 50 | 23 |
| 1.51 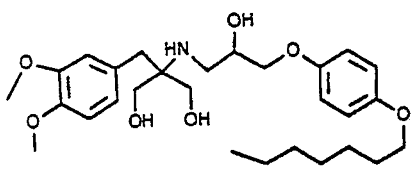 | 100 | 100 |
| | 50 | 100 |
| | 10 | 75 |
| 1.51 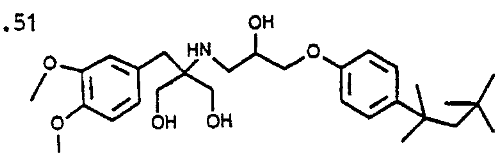 | 100 | 100 |
| | 50 | 100 |
| | 10 | 75 |

Fortsetzung Tabelle 1

| Präparat | Konzentration (µm/ml) | % Hemmung der Phospholipase |
|---|---|---|
| 1.52 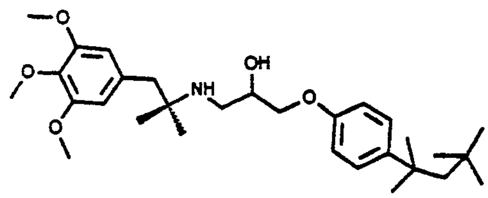 | 100 | 100 |
| | 50 | 100 |
| | 10 | 67 |
| 1.53 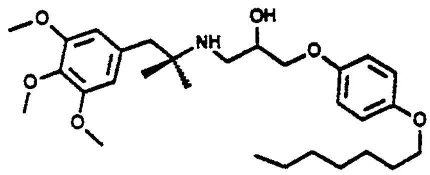 | 50 | 100 |
| | 10 | 100 |
| 1.54 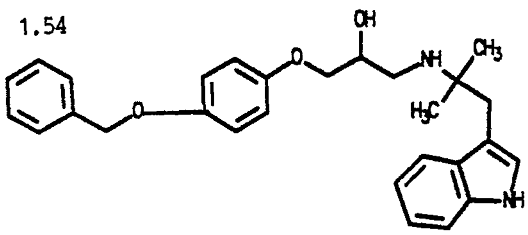 | 100 | 100 |
| | 50 | 100 |
| 1.55 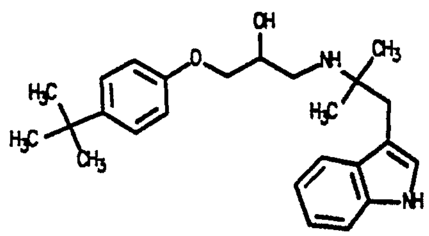 | 100 | 100 |
| | 50 | 100 |
| 1.56 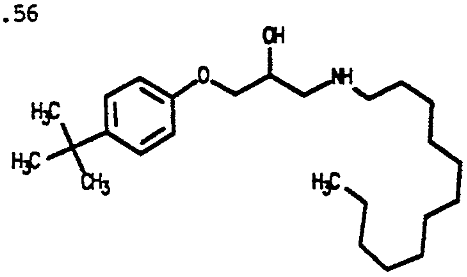 | 100 | 100 |
| | 50 | 100 |

Fortsetzung Tabelle 1

| Präparat | | Konzentration (µm/ml) | % Hemmung der Phospholipase |
|---|---|---|---|
| 1.57 | 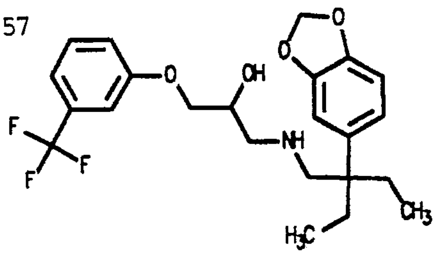 | 50 | 100 |
| 1.58 | 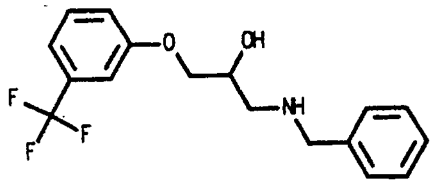 | 100<br>50 | 100<br>100 |
| 1.59 | 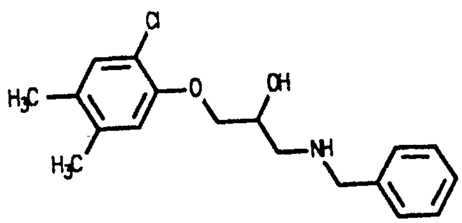 | 100<br>50 | 100<br>100 |
| 1.60 | 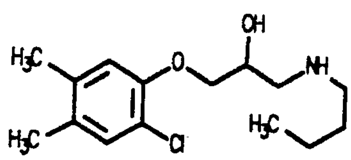 | 100<br>50 | 100<br>100 |
| 1.61 | 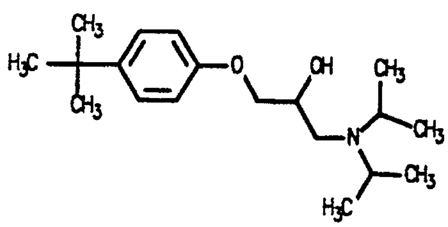 | 100<br>50 | 100<br>60 |

Fortsetzung Tabelle 1

| Präparat | Konzentration (µm/ml) | % Hemmung der Phospholipase |
|---|---|---|
| 1.62 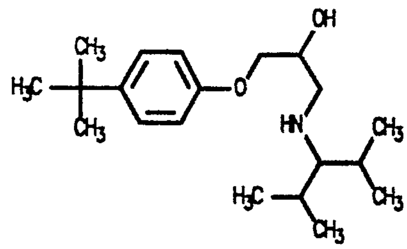 | 100 | 100 |
| | 50 | 100 |
| 1.63 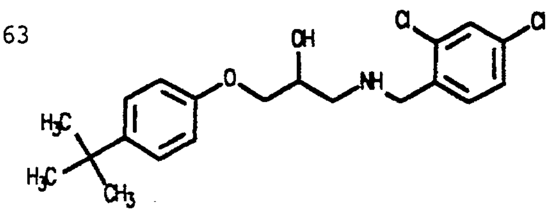 | 100 | 100 |
| | 50 | 100 |
| | 10 | 100 |
| | 5 | 34 |
| 1.64 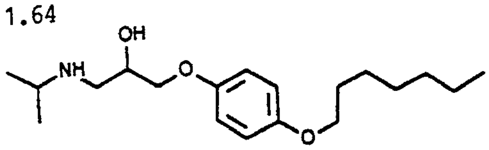 | 100 | 100 |
| | 50 | 100 |
| | 10 | 30 |
| 1.65 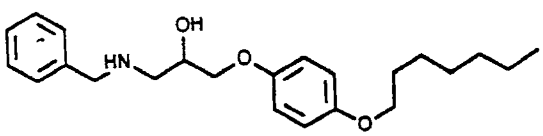 | 10 | 100 |
| | 5 | 100 |
| 1.66 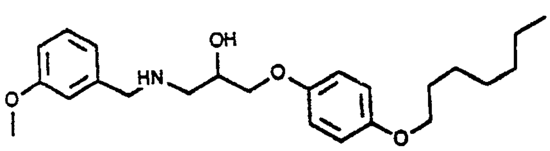 | 50 | 100 |
| | 10 | 100 |
| | 5 | 40 |
| 1.67 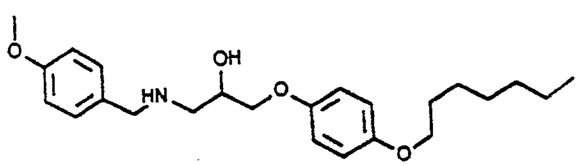 | 10 | 100 |
| | 5 | 100 |

Fortsetzung Tabelle 1

| Präparat | Konzentration (µm/ml) | % Hemmung der Phospholipase |
|---|---|---|
| 1.68 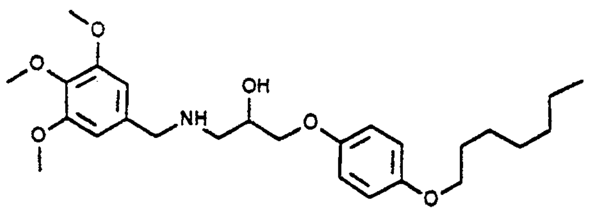 | 100<br>50<br>10 | 100<br>100<br>30 |
| 1.69 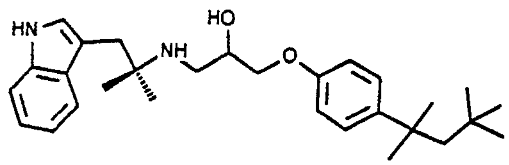 | 100<br>50 | 100<br>100 |

Als Beispiele für die systemische in vivo-Wirksamkeit der Verbindungen dient die systemische Candida albicans Infektion bei der Maus.

Ziel dieser Methode ist es, den Effekt von Präparaten auf eine systemische Candida albicans Infektion zu ermitteln. Die Infektion führt innerhalb von 2-4 Tagen zum Tod der Tiere.

Beschreibung der Methode: Albino Mäuse (NMRI, Männchen, 15-20 g Körpergewicht) werden intravenös mit Candida albicans Hefezellen infiziert (Stamm 200/175), die frisch auf Malzagar vorkultiviert wurden und in physiologischer Kochsalzlösung suspendiert wurden. Die Infektionsdosis, die in dem angegebenen Zeitrahmen zur Lethalität führt, wurde photometrisch eingestellt und enthielt eine Million Hefezellen pro Maus.

Die zu prüfenden Verbindungen werden entweder alleine oder in Kombination mit einem Standardantimykotikum verabreicht. Im durchgeführten Test wurde ein Kombinationspräparat verwendet, wobei als Standardantimykotikum Fluconazol (Fa. Pfizer) diente. Applikationswege sind je nach Substanzgruppe unterschiedlich, wobei der oralen Applikation der Vorzug gegeben wird. Die Mäuse werden 4 Wochen beobachtet, die Mortalität wird täglich registriert, und die Überlebenszeiten der einzelnen Gruppen werden gemittelt und untereinander verglichen. Gruppengröße beträgt durchschnittlich 10 Tiere.

Der Vergleich der Mittelwerte geschieht mit dem Students T Test.

Wie Tabelle 2 zeigt, verlängert sich beispielsweise bei Gabe von Verbindung 1.16 (14 x 50 mg/kg p.o. 1 x täglich) die Überlebenszeit von mit candida infizierten Mäusen um 65 % (Die Überlebenszeit der mittels Fluconazol-Monotherapie behandelten Tiere wurde als 100 definiert, worauf sich für die Kombinationstherapie 165 ergaben).

Tabelle 2 Überlebenszeiten von mit Candida infizierten Mäusen bei Kombinationstherapie mit Fluconazol

| Verbindung | | Überlebenszeit (bez. auf Fluconazol = 100 % ) |
|---|---|---|
| Fluconazol | | 100 % |
| Fluconazol | + Verbd. 1.7 | 127 % |
| Fluconazol | + " 1.9 | 131 % |
| Fluconazol | + " 1.10 | 149 % |
| Fluconazol | + " 1.16 | 165 % |

Dosierungen:
Fluconazol jeweils 8 x p.o. 50 mg/kg/Tag
β-Hydroxy-ethylamin jeweils 14 x p.o. 50 mg/kg/Tag

Die pilzabtötende, antimykotische Wirksamkeit der Verbindungen wurde an einigen Beispielen (s. Tabelle 3) anhand der prozentualen Abtötung von Trichophyton mentagrophytes in vitro verifiziert (Standardpräparat Rilopirox als Vergleichssubstanz).

Tabelle 3

| Verbindung | µg/ml | | | | | |
|---|---|---|---|---|---|---|
| | 80 | 40 | 20 | 10 | 5 | 2,5 |
| 1.1 | 100 | 100 | 99,8 | 99.4 | 98,7 | 80,8 |
| 1.2 | 100 | 100 | 100 | 100 | 98,7 | 92,3 |
| 1.3 | 100 | 100 | 99,8 | 98,9 | 74,9 | 52,7 |
| 1.17 | 100 | 100 | 100 | 99,8 | 87,1 | 73,4 |
| 1.18 | 100 | 100 | 100 | 100 | 95,7 | 79,3 |
| Rilopirox | 100 | 100 | 100 | 100 | 96,2 | 94,1 |

(Werte in %-Abtötung von Trichophyton mentagrophytes nach 14 h in aqua dest.)

Beispiele für die fungizide Wirksamkeit

Weinsämlinge wurden ca. 6 Wochen nach der Aussaat mit wäßrigen Suspensionen der zu untersuchenden Verbindungen gleichmäßig benetzt. Nach dem Antrocknen des Spritzbelages wurden die Pflanzen mit einer Sporensuspension von Plasmopara viticola inokuliert und für kurze Zeit in eine Klimakammer (23 °C und 80-90 % rel. Luftfeuchte) gestellt. Nach einer Inkubationszeit von 7 Tagen wurden die Pflanzen zur Sporulation des Pilzes erneut für einige Stunden in die Klimakammer gestellt. Anschließend erfolgte die Auswertung des Befalls. Der Befallsgrad wird ausgedrückt in % befallener Blattfläche im Vergleich zu den unbehandelten, infizierten Kontrollpflanzen und ist in Tabelle 4 wiedergegeben.

Tabelle 4

| Verbindungen gemäß Beispiel | mit Plasmopara viticola befallene Blattfläche in % bei ppm Wirkstoff (mg Wirkstoff/Liter Spritzbrühe) | | |
|---|---|---|---|
| | 500 | 250 | 125 |
| 1.32 | 0 | 0 | 0 |
| 1.37 | 0 | 0 | 0 |
| unbehandelte, infizierte Kontrollpflanzen | | 100 | |

Es zeigt sich, daß die mit den erfindungsgemäßen Verbindungen behandelten Pflanzen vom Pilz nicht befallen werden.

## Patentansprüche

**1.** Verwendung einer Verbindung der Formel III

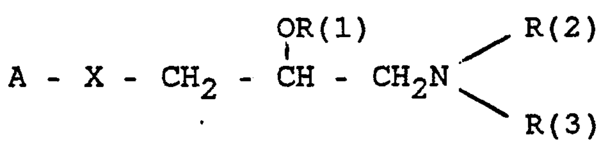

(III)

und von ihren Salzen mit pharmazeutisch akzeptablen Säuren sowie von diese Verbindungen III enthaltenden pharmazeutischen Zubereitungen zur Herstellung eines Medikaments zur Vorbeugung gegen und zur Behandlung von Pilzerkrankungen, wobei die Reste in Formel III folgende Bedeutung haben:

R(1) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), Benzyl, [unsubstituiert oder ein- oder mehrfach substituiert durch F, Cl, Br, $CF_3$, ($C_1$-$C_4$)-Alkyl (geradkettig oder verzweigt, $OCH_3$, O-Phenyl oder Phenyl], C(O)-($C_1$-$C_8$)-Alkyl (geradkettig oder verzweigt), C(O)-phenyl

R(2) H, ($C_1$-$C_{18}$)-Alkyl (geradkettig oder verzweigt), ($C_1$-$C_6$)-Alkyl-oxy-($C_1$-$C_6$)-Alkyl (geradkettig oder verzweigt), ($C_1$-$C_6$)-Alkyl-oxy-phenyl (geradkettig oder verzweigt), ($C_2$-$C_{18}$)-Alkenyl (geradkettig oder verzweigt, ein- oder mehrfach ungesättigt), ($C_3$-$C_{12}$)-Cycloalkyl (mono-, bi- oder multicyclisch), Phenyl-($C_1$-$C_6$)-Alkyl (geradkettig oder verzweigt, wobei die Alkylkette unsubstituiert oder mit OH ein- oder zweifach substituiert ist), Phenyl-($C_2$-$C_6$)-Alkenyl (geradkettig oder verzweigt, ein- oder mehrfach ungesättigt), Diphenyl-($C_1$-$C_6$)-Alkyl (geradkettig oder verzweigt), Phenyl, wobei die Phenylsysteme unsubstituiert oder ein- oder mehrfach substituiert sind durch Substituenten aus der Gruppe F, Cl, Br, ($C_1$-$C_{10}$)-Alkyl (geradkettig oder verzweigt), ($C_3$-$C_{10}$)-Cycloalkyl, OH, SH ($C_1$-$C_{10}$)-Alkoxy (geradkettig oder verzweigt), ($C_1$-$C_4$)-Alkylendioxy, Dimethylaminoethoxy, ($C_1$-$C_4$)-Alkoxycarbonylmethoxy, Phenoxy, Phenyl, Benzyl, Phenethyl, Thiophenyl, $C_nF_{2n+1}$ mit n gleich 1-6),

oder

R(2) ist ein Indol-3-yl-($C_1$-$C_4$)-Alkylrest (geradkettig oder verzweigt), Thienyl, Thienylmethyl, wobei der Thienylrest unsubstituiert oder mit F, Cl, ($C_1$-$C_4$)-Alkyl, O($C_1$-$C_4$)-Alkyl (geradkettig oder verzweigt) substituiert ist,

R(3) ist wie R(2) definiert, wobei R(2) und R(3) jeweils gleiche oder verschiedene Bedeutung

aufweisen, oder

R(2) bildet mit R(3) eine Kette $-(CH_2)_m-$ mit m gleich 4-6, in welcher eine $CH_2$-Gruppe durch Sauerstoff, Schwefel oder Stickstoff ersetzt sein kann, wobei das zusätzliche N als weiteren Bindungspartner ein H-Atom, eine $CH_3$, Phenyl, Benzyl oder Phenethylgruppe trägt, und

A $(C_1-C_{18})$-Alkyl (geradkettig oder verzweigt), $(C_2-C_{18})$-Alkenyl (geradkettig oder verzweigt, ein- oder mehrfach ungesättigt) oder eine Gruppe der Formel II ist,

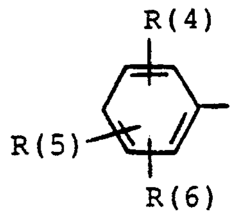

II

worin die Reste R(4), R(5) und R(6) die folgende Bedeutung haben:

R(4) H, $(C_1-C_{18})$-Alkyl (geradkettig oder verzweigt), $(C_2-C_{18})$-Alkenyl (geradkettig oder verzweigt, ein- oder mehrfach ungesättigt), $(C_3-C_{20})$-Cycloalkyl,[mono-,bi- oder multicyclisch, unsubstituiert oder ein- oder zweifach mit $(C_1-C_4)$-Alkyl (geradkettig oder verzweigt), $(C_1-C_4)$-Alkoxy (geradkettig oder verzweigt), $C_nF_{2n+1}$ mit n gleich 1-4,F, Cl, Br, OH substituiert], Y-$(C_1-C_{20})$-Alkyl (geradkettig oder verzweigt), Y-$(C_2-C_{18})$-Alkenyl (geradkettig oder verzweigt ein- oder mehrfach ungesättigt), Y-$(C_3-C_{20})$-Cycloalkyl (mono-, bi- oder multicyclisch, unsubstituiert oder wie oben angegeben substituiert), Phenyl, Y-Phenyl, Phenyl-$(C_1-C_4)$-Alkyl (geradkettig oder verzweigt), Phenyl-$(C_1-C_4)$-Alkoxy (geradkettig oder verzweigt), Biphenylyl, F, Cl, Br, $C_nF_{2n+1}$ (mit n gleich 1-8), $CCl_3$, YH, Naphthyl, CN, $NO_2$, wobei die Phenylsysteme unsubstituiert sind oder ein- oder zweifach substituiert durch F, Cl, $CF_3$, $(C_1-C_4)$-Alkyl (geradkettig der verzweigt), $(C_1-C_4)$-Alkoxy (geradkettig oder verzweigt),

mit Y gleich Sauerstoff oder Schwefel, SO, $SO_2$,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)p$-Kette mit p gleich 3 oder 4, und

R(6) H, $(C_1-C_{15})$-Alkyl, (geradkettig oder verzweigt), $(C_2-C_{15})$-Alkenyl (geradkettig oder verzweigt, ein- oder mehrfach ungesättigt), Y-$(C_1-C_{15})$-Alkyl (geradkettig oder versweigt), Y-$(C_2-C_{15})$-Alkenyl (geradkettig oder verzweigt, ein- oder mehrfach ungesättigt), Phenyl, Y-Phenyl, Benzyl, Biphenylyl, F, Cl, Br, I, $C_nF_{2n+1}$ (mit n gleich 1-8), $CCl_3$, Naphthyl, YH,

X Sauerstoff, Schwefel.

**2.** Verwendung einer Verbindung der Formel III nach Anspruch 1, in welcher die Reste folgende Bedeutung haben:

R(1) H, $(C_1-C_8)$-Alkyl (geradkettig oder verzweigt), $(C_3-C_8)$-Alkenyl (geradkettig oder verzweigt, ein- oder zweifach ungesättigt), Benzyl [unsubstituiert oder ein- oder zweifach substituiert durch F, Cl, $CF_3$, $(C_1-C_4)$-Alkyl (geradkettig oder verzweigt), $OCH_3$], C(O)-$(C_1-C_6)$-Alkyl (geradkettig oder verzweigt),

R(2) H, $(C_1-C_{16})$-Alkyl (geradkettig oder verzweigt), $(C_1-C_6)$-Alkyl-oxy-$(C_1-C_6)$-Alkyl (geradkettig oder verzweigt), $(C_1-C_6)$-Alkyl-oxy-phenyl, $(C_2-C_{16})$-Alkenyl (geradkettig oder verzweigt, ein- oder mehrfach ungesättigt), $(C_3-C_{10})$-Cycloalkyl, (mono-, bi- oder multicyclisch), Phenyl-$(C_1-C_6)$-Alkyl (geradkettig oder verzweigt), Diphenyl-$(C_1-C_6)$-Alkyl (geradkettig oder verzweigt), Phenyl, wobei die Phenylsysteme unsubstituiert oder ein- oder zweifach substituiert sind durch Substituenten aus der Gruppe F, Cl, $(C_1-C_4)$-Alkyl (geradkettig oder verzweigt), $(C_3-C_8)$-Cycloalkyl, $(C_1-C_8)$-Alkoxy (geradkettig oder verzweigt), $(C_1-C_2)$-Alkylendioxy, $(C_1-C_4)$-Alkoxycarbonylmethoxy, Phenyl, Benzyl, Phenethyl, Thiophenyl,

oder

R(2) ist ein Indol-3-yl-$(C_1-C_4)$-Alkylrest (geradkettig oder verzweigt), Thienylmethyl, wobei der Thienylrest unsubstituiert ist oder substituiert ist mit Cl, $(C_1-C_4)$-Alkyl (geradkettig oder verzweigt), O$(C_1-C_4)$-Alkyl (geradkettig oder verzweigt),

R(3) ist wie R(2) definiert, wobei R(2) und R(3) jeweils gleiche oder verschiedene Bedeutung aufweisen, oder

R(2) bildet mit R(3) eine Kette $-(CH_2)_m-$ mit m gleich 4-6, in welcher eine $CH_2$-Gruppe durch

Sauerstoff, Schwefel oder Stickstoff ersetzt sein kann, wobei das zusätzliche N als weiteren Bindungspartner ein H-Atom, eine $CH_3$, Phenyl, Benzyl oder Phenethylgruppe trägt, und

A $(C_1-C_{16})$-Alkyl (geradkettig oder verzweigt), $(C_3-C_{16})$-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, $(C_1-C_{16})$-Alkyl (geradkettig oder verzweigt), $(C_2-C_{16})$-Alkenyl (geradkettig oder verzweigt, ein- oder mehrfach ungesättigt), $(C_3-C_{12})$-Cycloalkyl [mono-, bi- oder multicyclisch, unsubstituiert oder einfach mit $(C_1-C_4)$-Alkyl (geradkettig oder verzweigt) oder $(C_1-C_4)$-Alkoxy (geradkettig oder verzweigt) substituiert], Y-$(C_1-C_{16})$-Alkyl (geradkettig oder verzweigt), Y-$(C_3-C_{16})$-Alkenyl (geradkettig oder verzweigt, ein- oder mehrfach ungesättigt), Y-$(C_3-C_{12})$-Cycloalkyl (mono-, bi- oder multicyclisch, unsubstituiert oder wie oben angegeben subsituiert), Phenyl, Y-Phenyl, Phenyl-$(C_1-C_2)$-Alkyl, Phenyl-$(C_1-C_4)$-Alkoxy) (geradkettig oder verzweigt), Biphenylyl, F, Cl, $C_nF_{2n+1}$ (mit n gleich 1-4), $CCl_3$, Naphthyl, CN, wobei die Phenylsysteme unsubstituiert sind oder ein- oder zweifach durch F, Cl, $CF_3$, $(C_1-C_4)$-Alkyl, $(C_1-C_4)$-Alkoxy

mit Y gleich Sauerstoff oder Schwefel,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)$p-Kette mit p gleich 3 oder 4, und

R(6) H, $(C_1-C_{12})$-Alkyl (geradkettig oder verzweigt), $(C_3-C_{12})$-Alkenyl (geradkettig oder verzweigt, ein- oder mehrfach ungesättigt), Y-$(C_1-C_{12})$-Alkyl (geradkettig oder verzweigt), Y-$(C_3-C_{12})$-Alkenyl (geradkettig oder verzweigt, ein- oder mehrfach ungesättigt), Phenyl, Y-Phenyl, Benzyl, Biphenylyl, F, Cl, Br, $C_nF_{2n+1}$ (mit n gleich 1-4), $CCl_3$, Naphthyl,

X Sauerstoff, Schwefel.

**3.** Verwendung einer Verbindung der Formel III nach Anspruch 1, wobei die Reste in Formel III folgende Bedeutung haben:

R(1) H, C(O)-$(C_1-C_4)$-Alkyl (geradkettig oder verzweigt), Benzyl,

R(2) H, $(C_1-C_{12})$-Alkyl (geradkettig oder verzweigt), $(C_1-C_6)$-Alkyl-oxy-$(C_1-C_6)$-Alkyl, $(C_3-C_{12})$-Alkenyl (geradkettig oder verzweigt, ein- oder mehrfach ungesättigt), $(C_3-C_{10})$-Cycloalkyl (mono-, bi- oder multicyclisch), Phenyl-$(C_1-C_6)$-Alkyl (geradkettig oder verzweigt), Diphenyl-$(C_1-C_6)$-Alkyl (geradkettig oder verzweigt), Phenyl, wobei die Phenylsysteme unsubstituiert oder ein- oder zweifach substituiert sind durch Substituenten aus der Gruppe $CF_3$, F, Cl, $(C_1-C_4)$-Alkyl (geradkettig oder verzweigt), $(C_1-C_8)$-Alkoxy (geradkettig oder verzweigt), $(C_1-C_2)$-Alkylendioxy, Phenyl, Benzyl, Phenethyl),

oder

R(2) ist ein Indol-3-yl$(C_1-C_4)$-Alkylrest (geradkettig oder verzweigt), Thienylmethyl, wobei der Thienylrest unsubstituiert oder mit Cl, $(C_1-C_4)$-Alkyl, $O(C_1-C_4)$-Alkyl substituiert ist

R(3) ist H,

R(2) bildet mit R(3) eine Kette $-(CH_2)_m-$ mit m gleich 4-6, in welcher eine $CH_2$-Gruppe durch Sauerstoff, Schwefel oder Stickstoff ersetzt sein kann, wobei N als weiteren Bindungspartner ein H-Atom, eine $CH_3$, Phenyl,Benzyl oder Phenethylgruppe trägt, und

A $(C_1-C_{12})$-Alkyl (geradkettig oder verzweigt), $(C_3-C_{12})$-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, $(C_1-C_{16})$-Alkyl (geradkettig oder verzweigt), $(C_2-C_{12})$-Alkenyl (geradkettig oder verzweigt, ein- oder mehrfach ungesättigt), $(C_3-C_{12})$-Cycloalkyl (mono-, bi- oder multicyclisch und unsubstituiert), Y-$(C_1-C_{12})$-Alkyl (geradkettig oder verzweigt), Y-$(C_3-C_{12})$-Alkenyl (geradkettig oder verzweigt, ein- oder mehrfach ungesättigt), Y-$(C_3-C_{12})$-Cycloalkyl (mono-, bi- oder multicyclisch und unsubstituiert), Phenyl, Y-Phenyl, Phenyl-$(C_1-C_2)$-Alkyl, Phenyl-$(C_1-C_4)$-Alkoxy (geradkettig oder verzweigt), Biphenyl, F, Cl, Naphthyl, wobei die Phenylsysteme unsubstituiert sind oder ein- oder zweifach substituiert durch F, Cl, $CF_3$, $(C_1-C_4)$-Alkyl, $(C_1-C_4)$-Alkoxy (geradkettig oder verzweigt)

mit Y gleich Sauerstoff oderSchwefel und

R(5) wie R(4) definiert, wobei R(4) und R(5) gleich oder verschieden sind, und

R(6) H, $(C_1-C_{10})$-Alkyl (geradkettig oder verzweigt), $(C_3-C_{10})$-Alkenyl (geradkettig oder verzweigt, ein- oder mehrfach ungesättigt), Y-$(C_1-C_{10})$-Alkyl (geradkettig oder verzweigt, ein- oder mehrfach ungesättigt), Phenyl, Y-Phenyl, Benzyl, F, Cl, $CF_3$,

X Sauerstoff.

4. Verwendung einer Verbindung der Formel III nach Anspruch 1, wobei die Reste in Formel III folgende Bedeutung haben:

R(1) H, C(O)-$CH_3$, Benzyl

R(2) H, ($C_1$-$C_{10}$)-Alkyl (geradkettig oder verzweigt), ($C_1$-$C_3$)-Alkyl-oxy-($C_1$-$C_3$)-Alkyl, ($C_3$-$C_{10}$)-Alkenyl (geradkettig oder verzweigt ein- oder mehrfach ungesättigt), ($C_1$-$C_{10}$)-Cycloalkyl, (mono-, bi- oder multicyclisch), Phenyl-($C_1$-$C_4$)-Alkyl (geradkettig oder verzweigt), Diphenyl-($C_1$-$C_4$)-Alkyl (geradkettig oder verzweigt), Phenyl, wobei die Phenylsysteme unsubstutiert oder ein- oder zweifach substituiert sind durch Substituenten aus der Gruppe F, Cl, ($C_1$-$C_4$)-Alkyl (geradkettig oder verzweigt), ($C_1$-$C_6$)-Alkoxy (geradkettig oder verzweigt), ($C_1$-$C_2$)-Alkylendioxy, Phenyl, Benzyl, Phenethyl,

oder

R(2) ist ein Indol-3-yl-($C_1$-$C_4$)-Alkylrest (geradkettig oder verzweigt), Thienylmethyl, wobei der Thienylrest unsubstituiert oder mit Cl, ($C_1$-$C_4$)-Alkyl, O($C_1$-$C_4$)-Alkyl substituiert ist,

und

R(3) H

und

A ($C_1$-$C_{12}$)-Alkyl (geradkettig oder verzweigt), ($C_3$-$C_{10}$)-Alkenyl (geradkettig oder verzweigt, ein- oder mehrfach ungesättigt) oder eine Gruppe der Formel II ist,

worin die Reste R(4), R(5) und R(6) die folgende Bedeutung haben:

R(4) H, ($C_1$-$C_{12}$)-Alkyl (geradkettig oder verzweigt), ($C_2$-$C_{12}$)-Alkenyl (geradkettig oder verzweigt, ein- oder mehrfach ungesättigt), ($C_3$-$C_{10}$)-Cycloalkyl (mono-, bi- oder multicyclisch und unsubstituiert), Y-($C_1$-$C_{12}$)-Alkyl (geradkettig oder verzweigt), Y-($C_3$-$C_{12}$)-Alkenyl (geradkettig oder verzweigt, ein- oder mehrfach ungesättigt), Y-($C_3$-$C_{10}$)-Cycloalkyl (mono-, bi- oder multicyclisch und unsubstituiert), Phenyl, Y-Phenyl, Benyl, Benzyloxy, Biphenylyl, F, Cl, $CF_3$, wobei die Phenylsysteme unsubstituiert sind oder ein- oder zweifach substituiert durch F, Cl, $CF_3$, ($C_1$-$C_4$)-Alkyl (geradkettig oder verzweigt), ($C_1$-$C_4$)-Alkoxy (geradkettig oder verzweigt)

mit Y gleich Sauerstoff und

R(5) H, ($C_1$-$C_{12}$)-Alkyl (geradkettig oder verzweigt, ($C_2$-$C_{12}$)-Alkenyl (geradkettig oder verzweigt, ein- oder zweifach ungesättigt), ($C_3$-$C_6$)-Cycloalkyl, Y-($C_1$-$C_{12}$)-Alkyl (geradkettig oder verzweigt), Y-($C_3$-$C_6$)-Cycloalkyl,Phenyl, Y-Phenyl, Benzyl, F, Cl, $CF_3$ und

R(6) gleich H

X gleich Sauerstoff.

5. Verwendung einer Verbindung der Formel I

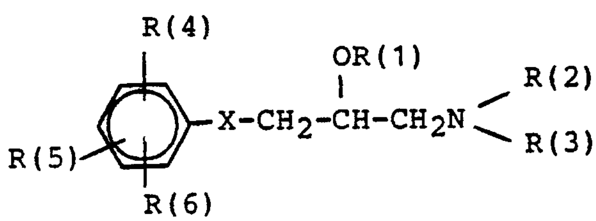

I,

und ihrer Salze mit pharmazeutisch akzeptablen Säuren zur Herstellung eines Medikaments zum Behandeln von Pilzerkrankungen, in welchen Verbindungen bedeuten:

R(1) H, ($C_1$-$C_{10}$)-Alkyl (geradkettig oder verzweigt),($C_2$-$C_{10}$)-Alkenyl (geradkettig oder verzweigt, ein- oder mehrfach ungesättigt), Benzyl (unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl)

R(2) H, ($C_1$-$C_{10}$)-Alkyl (geradkettig oder verzweigt), ($C_2$-$C_{10}$)-Alkenyl (geradkettig oder verzweigt, ein- oder mehrfach ungesättigt), ($C_3$-$C_{10}$)-Cycloalkyl, Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-Alkyl (geradkettig oder verzweigt), Phenyl, wobei das Phenylsystem unsubstituiert ist oder ein- oder mehrfach substituiert ist durch Substituenten aus der

Gruppe F, Cl, Br, ($C_1$-$C_4$)-Alkyl (geradkettig oder verzweigt), ($C_3$-$C_8$)-Cycloalkyl, OH, SH, ($C_1$-$C_{10}$)-Alkoxy (geradkettig oder verzweigt), Phenyl, Benzyl, Phenethyl, Thiophenyl, $C_nF_{2n+1}$ mit n gleich 1-6),

R(3) wie R(2) definiert ist, wobei R(2) und R(3) jeweils gleiche oder verschiedene Bedeutung aufweisen,

oder

R(2) mit R(3) eine Kette -$(CH_2)_m$- mit m gleich 4-6 bildet, in welcher eine $CH_2$-Gruppe durch Sauerstoff, Schwefel oder Stickstoff ersetzt sein kann, wobei N als weiteren Bindungspartner ein H-Atom, eine $CH_3$, Phenyl-, Benzyl oder Phenethylgruppe trägt,

und

R(4) H, ($C_1$-$C_{15}$)-Alkyl (geradkettig oder verzweigt), $C_2$)-$C_{15}$)-Alkenyl (geradkettig oder verzweigt, ein- oder mehrfach ungesättigt), ($C_3$-$C_{20}$)-Cycloalkyl [mono-, bi-oder multicyclisch, unsubstituiert oder ein oder zweifach mit ($C_1$-$C_4$)-Alkyl (geradkettig oder verzweigt), ($C_1$-$C_4$)-Alkoxy (geradkettig oder verzweigt), $C_nF_{2n+1}$ (mit n gleich 1-4), F, Cl, Br, OH substituiert], Y-($C_1$-$C_{15}$)-Alkyl (geradkettig oder verzweigt), Y-($C_2$-$C_{15}$)-Alkenyl (geradkettig oder verzweigt, ein- oder mehrfach ungesättigt), Y-($C_3$-$C_{20}$)-Cycloalkyl (mono-, bi- oder multicyclisch, unsubstituiert oder wie oben angegeben substituiert), Phenyl, Y-Phenyl, Phenyl-($C_1$-$C_2$)-Alkyl, Biphenylyl, F, Cl, Br, I, $C_nF_{2n+1}$ (mit n gleich 1-8), $CCl_3$, YH, Naphthyl, CN,-$NO_2$ mit Y gleich Sauerstoff oder Schwefel

und

R(5) wie R(4) definiert, wobei R(4) und R(5) gleich oder verschieden sind,

und

R(4) mit R(5), für den Fall, daß die Substituenten an benachbarten Positionen am Phenylring gebunden sind, gemeinsam eine $(CH_2)_p$-Kette mit p gleich 3 oder 4 bilden können,

und

R(6) H, ($C_1$-$C_{15}$)-Alkyl (geradkettig oder verzweigt), ($C_2$-$C_{10}$)-Alkenyl (geradkettig oder verzweigt, ein- oder mehrfach ungesättigt), Y-($C_1$-$C_{15}$)-Alkyl (geradkettig oder verzweigt), Y-($C_2$-$C_{15}$)-Alkenyl (geradkettig oder verzweigt, ein oder mehrfach ungesättigt), Phenyl, Y-Phenyl, Benzyl, Biphenylyl, F, Cl, Br, I, $C_nF_{2n+1}$ (mit n gleich 1-8), $CCl_3$, Naphthyl, YH

Y = Sauerstoff, Schwefel

X = Sauerstoff, Schwefel, SO, $SO_2$.

6. Verwendung von Verbindungen I nach Anspruch 5, in denen bedeuten:

R(1) H, ($C_1$-$C_8$)-Alkyl (geradkettig oder verzweigt),($C_3$-$C_{10}$)-Alkenyl (geradkettig oder verzweigt, ein- oder mehrfach ungesättigt), Benzyl (unsubstituiert oder einfach oder zweifach substituiert durch F, Cl, $CF_3$, ($C_1$-$C_4$)-Alkyl (geradkettig oder verzweigt), $OCH_3$,

R(2) H, ($C_1$-$C_8$)-Alkyl (geradkettig oder verzweigt), ($C_3$-$C_{10}$)-Alkenyl (geradkettig oder verzweigt, ein- oder mehrfach ungesättigt), Phenyl-($C_1$-$C_6$)-Alkyl (geradkettig oder verzweigt), Diphenyl-($C_1$-$C_6$)-Alkyl (geradkettig oder verzweigt), Phenyl, wobei das Phenylsystem unsubstituiert ist oder ein- oder zweifach substituiert ist durch Substituenten aus der Gruppe OH; F, Cl, Br, ($C_1$-$C_4$)-Alkyl (geradkettig oder verzweigt), ($C_1$-$C_4$)-Alkoxy (geradkettig oder verzweigt), Phenyl, Benzyl, Phenethyl,

R(3) wie R(2) definiert ist, wobei R(2) und R(3) jeweils gleiche oder verschiedene Bedeutung aufweisen,

oder

R(2) mit R(3) eine Kette $(CH_2)_m$- mit m gleich 4-6 bildet, in welcher eine $CH_2$-Gruppe durch ein Sauerstoff oder Stickstoff-Atom ersetzt sein kann, wobei N als weiteren Bindungspartner ein H-Atom, eine $CH_3$, Phenyl-, Benzyl oder Phenethylgruppe trägt,

und

R(4) H, ($C_1$-$C_{15}$)-Alkyl (geradkettlg oder verzweigt, ($C_2$-$C_{15}$)-Alkenyl (geradkettig oder verzweigt, ein- oder mehrfach ungesättigt), ($C_3$-$C_{15}$)-Cycloalkyl [mono, bi- oder multicyclisch, unsubstituiert oder einfach mit ($C_1$-$C_4$)-Alkyl (geradkettig oder verzweigt), ($C_1$-$C_4$)-Alkoxy (geradkettig oder verzweigt), substituiert], Y-($C_1$-$C_{10}$)-Alkyl (geradkettig oder verzweigt), Y-($C_2$-$C_{10}$)Alkenyl (geradkettig oder verzweigt, ein- oder mehrfach ungesättigt), Y-($C_3$-$C_{15}$)-Cycloalkyl (mono-, bi- oder multicyclisch, unsubstituiert oder wie oben angegeben substituiert), Phenyl, Y-Phenyl, Phenyl-($C_1$-$C_2$)-Alkyl, Biphenylyl, F, Cl, $C_nF_{2n+1}$ (mit n gleich 1-4), $CCl_3$, Naphthyl, CN, mit Y gleich Sauerstoff oder Schwefel

und

R(5) wie R(4) definiert ist, wobei R(4) und R(5) gleich oder verschieden sind,

und

R(4) mit R(5) für den Fall, daß die Substituenten an benachbarten Positionen am Phenylring gebunden sind, gemeinsam eine $(CH_2)_p$-Kette mit p = 3 oder 4 bilden können,

und

R(6) H, $(C_1$-$C_{10})$-Alkyl (geradkettig oder verzweigt), Y-$(C_1$-$C_{10})$-Alkyl (geradkettig oder verzweigt, Y-$(C_2$-$C_{10})$-Alkyenyl (geradkettig oder verzweigt), ein- oder mehrfach ungesättigt), Phenyl, Benzyl, Biphenylyl, F, Cl, Br, $C_nF_{2n+1}$

Y = Sauerstoff, Schwefel

X = Sauerstoff, Schwefel.

7. Verwendung von Verbindungen I nach Anspruch 5, in denen bedeuten:

R(1) H, $(C_1$-$C_8)$-Alkyl (geradkettig oder verzweigt), Benzyl (unsubstituiert oder ein- oder zweifach substituiert durch F, Cl, $CF_3$, $(C_1$-$C_4)$-Alkyl (geradkettig oder verzweigt), $OCH_3$,

R(2) H, $(C_1$-$C_8)$-Alkyl (geradkettig oder verzweigt), $(C_3$-$C_{10})$-Alkenyl (geradkettig oder verzweigt, ein- oder mehrfach ungesättigt), Phenyl-$(C_1$-$C_6)$-Alkyl (geradkettig oder verzweigt), Diphenyl-$(C_1$-$C_6)$-Alkyl (geradkettig oder verzweigt), Phenyl, wobei das Phenylsystem unsubstituiert ist oder ein- oder zweifach substituiert ist durch Substituenten aus der Gruppe OH, F, Cl, Br, $(C_1$-$C_4)$-Alkyl (geradkettig oder verzweigt), $(C_1$-$C_4)$-Alkoxy (geradkettig oder verzweigt), Phenyl, Benzyl,

R(3) H

oder

R(2) gemeinsam mit R(3) eine Kette $(CH_2)_m$- mit m gleich 4-5 bildet, in welcher eine $CH_2$-Gruppe durch ein Sauerstoff- oder N-Atom ersetzt sein kann, wobei N als weiteren Bindungspartner ein H-Atom, eine $CH_3$, Phenyl-, Benzyl oder Phenethylgruppe trägt,

und

R(4) H, $(C_1$-$C_{10})$-Alkyl (geradkettig oder verzweigt, $(C_2$-$C_{10})$-Alkenyl (geradkettig oder verzweigt, ein- oder mehrfach ungesättigt), $(C_3$-$C_{15})$-Cycloalkyl [mono-, bi- oder multicyclisch, wie z.B. Norbornyl, Adamantyl, Decahydronaphthalinyl]; Y-$(C_1$-$C_{10})$-Alkyl (geradkettig oder verzweigt), Y-$(C_2$-$C_{10})$-Alkenyl (geradkettig oder verzweigt, ein- oder mehrfach ungesättigt), Y-$(C_3$-$C_{15})$-Cycloalkyl (mono-, bi- oder multicyclisch), Phenyl, Y-Phenyl, Benzyl, Biphenylyl, F, Cl, $C_nF_{2n+1}$ (mit n gleich 1-4), $CCl_3$, Naphthyl, CN, mit Y gleich Sauerstoff oder Schwefel

und

R(5) wie R(4) definiert ist, wobei R(4) und R(5) gleich oder verschieden sind,

und

R(6) H, $(C_1$-$C_4)$-Alkyl (geradkettig oder verzweigt), Y-$(C_1$-$C_4)$-Alkyl (geradkettig oder verzweigt, F, Cl, $CF_3$

Y = Sauerstoff, Schwefel

X = Sauerstoff.

8. Verwendung von Verbindungen I nach Anspruch 5, bei denen die Substituenten bedeuten

R(1) H, Benzyl,

R(2) H, $(C_1$-$C_8)$-Alkyl (geradkettig oder verzweigt), Phenyl-$(C_1$-$C_4)$-Alkyl (geradkettig oder verzweigt), wobei das Phenylsystem unsubstituiert oder einfach substituiert ist durch F, Cl, $(C_1$-$C_4)$-Alkyl (geradkettig oder verzweigt), $(C_1$-$C_4)$-Alkoxy (geradkettig oder verzweigt),

R(3) H

und

R(4) H, $(C_1$-$C_{10})$-Alkyl (geradkettig oder verzweigt), $(C_2$-$C_{10})$-Alkenyl (geradkettig oder verzweigt, ein- oder mehrfach ungesättigt), $(C_3$-$C_{15})$-Cycloalkyl [mono-, bi- oder multicyclisch] Y-$(C_1$-$C_{10})$-Alkyl (geradkettig oder verzweigt), Y-$(C_3$-$C_{15})$-Cycloalkyl (mono-, bi- oder multicyclisch), Phenyl, Y-Phenyl, Benzyl, Biphenylyl, F, Cl, $C_nF_{2n+1}$ (mit n = 1-4), mit Y gleich Sauerstoff

und

R(5) H, $(C_1$-$C_6)$-Alkyl (geradkettig oder verzweigt), $(C_2$-$C_6)$-Alkenyl (geradkettig oder verzweigt, ein- oder mehrfach ungesättigt), $(C_3$-$C_6)$-Cycloalkyl Y-$(C_1$-$C_6)$-Alkyl (geradkettig oder verzweigt), Y-$(C_3$-$C_6)$-Cycloalkyl, Phenyl, Y-Phenyl, Benzyl, F, Cl, $CF_3$ mit Y gleich Sauerstoff und X gleich Sauerstoff,

R(6) H.

9. Verwendung einer Verbindung III nach Anspruch 1 zur Herstellung eines Medikaments zur Prophylaxe von Pilzerkrankungen.

10. Verwendung einer Verbindung I nach Anspruch 5 zur Herstellung eines Medikaments zur Prophylaxe von Pilzerkrankunen.